# EUROPEAN PATENT APPLICATION

(11) **EP 1 710 256 A1**
(43) Date of publication of application: **11.10.2006**
(21) Application number: 06015474.7
(22) Date of filing: 26.07.2000
(51) Int. Cl.: C07K 16/30, C07K 16/28, A01K 67/027, A61K 39/395, G01N 33/574, C12N 5/20, C07K 16/46, A61P 35/00

(54) **Human monoclonal antibodies to prostate specific membrane antigen**

(30) Priority: 29.07.1999 US 146285 P; 12.10.1999 US 158759 P; 09.03.2000 US 188087 P
(62) Divisional of application: 00950674.2
(71) Applicant: MEDAREX, INC., Annandale New Jersey 08801-0953 (US)
(72) Inventor: Deo, Yashwant, Audubon PA 19403 (US); Graziano, Robert, Frenchtown NJ 08825 (US); Hudson, Debra, Livermore CA 94550 (US); Tino, William T., Redmond WA 98052 (US); Holmes, Eric H., Bothell WA 98021 (US)
(74) Representative: Woods, Geoffrey Corlett

(57) **Abstract**

The invention provides an isolated human monoclonal antibody, or an antigen binding portion thereof, that specifically binds to PSMA, wherein the antibody or antigen binding portion thereof has one or more of the following characteristics:
a) a binding affinity constant to PSMA of at least about 10⁷ M⁻¹;
b) the ability to opsonize a cell expressing PSMA; or
c) the ability to inhibit growth or to mediate cytolysis of a cell expressing PSMA in the presence of human effector cells at a concentration of about 10 µg/ml or less *in vitro*.

## Description

### Background of the Invention

Prostate cancer is a highly prevalent cancer and a leading cause of morbidity and mortality among men. Treatments for prostate cancer include surgery, hormones, radiation and chemotherapy. There is little effective treatment for metastatic prostate disease, therefore the identification of genes and/or gene products that represent accurate diagnostic and prognostic markers, as well as targets for therapy is critical. Prostate specific antigen (PSA) is one such cancer marker, the values of which are useful in the clinical diagnosis and staging of prostate cancer. However, PSA cannot differentiate benign prostatic hyperplasia (BPH) from prostatitis or prostate cancer in the range of 4-10 ng/ml, thus necessitating a cytologic and/or histologic assessment to confirm the proper diagnosis (9).

Prostate specific membrane antigen (PSMA) is a 750 amino acid, type II transmembrane glycoprotein of approximately 110 kD that has 54% homology to the transferrin receptor. PSM' is an alternatively spliced form of PSMA which is localized in the cytoplasm. PSMA has 3 structural domains, including a 19 amino acid intracellular domain, a 24 amino acid transmembrane domain, and a 707 amino acid extracellular domain. The PSMA protein displays neurocarboxypeptidase and folate hydrolase activity, and therefore may be involved in the neuroendocrine regulation of prostate growth and differentiation (7).

Studies of PSMA expression indicate that it is a novel marker that is predominantly expressed by prostatic epithelial cells. Further, the expression of PSMA is increased in prostate cancer, especially in poorly differentiated, metastatic and hormone refractory carcinomas (8,11). Therefore PSMA represents a valuable diagnostic, prognostic and therapeutic target in prostate cancer. Low level expression of PSMA has also been observed in extraprostatic tissues such as the small bowel, salivary gland, duodenal mucosa, proximal renal tubules and brain (11).

PSMA in the brain is involved in the conversion of the neurotransmitter NAAG to HAA and free glutamate, which may be important in the pathogenesis of neurological disorders such as multiple sclerosis, amyotrophic lateral sclerosis, Alzheimer's disease and schizophrenia (12). PSMA expression is also observed in endothelial cells of capillary vessels in peritumoral and endotumoral areas of certain malignancies, including renal cell carcinomas and colon carcinomas, but not in blood vessels from normal tissues, suggesting that its expression may also be related to tumor angiogenesis (11).

### Summary of the Invention

The present invention provides isolated human monoclonal antibodies which specifically bind to prostate-specific membrane antigen (PSMA), as well as compositions containing one or a combination of such antibodies. In certain embodiments, the human antibody also is characterized by binding to PSMA with high affinity, and by inhibiting the growth and/or mediating cell killing (e.g., lysis or phagocytosis) of cells expressing PSMA (*in vitro* and *in vivo*) in the presence of human effector cells (e.g., polymorphonuclear cells, monocytes, macrophages and dendritic cells). Accordingly, the human monoclonal antibodies of the invention can be used as diagnostic or therapeutic agents in vivo and *in vitro.*

Isolated human antibodies of the invention encompass various antibody isotypes, such as IgG1, IgG2, IgG3, IgG4, IgM, IgA1, IgA2, IgAsec, IgD, and IgE. Typically, they include IgG1 (e.g., IgG1k) and IgM isotypes. The antibodies can be full-length (*e.g.,* an IgG1 or IgG4 antibody) or can include only an antigen-binding portion (*e.g.,* a Fab, F(ab')₂, Fv or a single chain Fv fragment). In one embodiment, the human antibodies are recombinant human antibodies. In another embodiment, the human antibodies are produced by a hybridoma which includes a B cell obtained from a transgenic non-human animal, e.g., a transgenic mouse, having a genome comprising a human heavy chain transgene and a human light chain transgene fused to an immortalized cell. In particular embodiments, the antibodies are produced by hybridomas referred to herein as 4A3, 7F12, 8A11,16F9 and 8C12.

In another embodiment, human anti-PSMA, antibodies of the present invention can be characterized by one or more of the following properties:
a) specificity for the PSMA;
b) a binding affinity to PSMA with an affinity constant of at least about 10⁷ M⁻¹, preferably about 10⁹ M⁻¹, and more preferably, about 10¹⁰ M⁻¹ to 10¹¹ M⁻¹ or higher;
c) an association constant (K_{assoc}) with PSMA of at least about 10³, more preferably about 10⁴ and most preferably about 10⁵ M⁻¹S⁻¹;
d) a dissociation constant (K_{dls}) from PSMA of about 10⁻³ s⁻¹, preferably about 10⁻⁴ s⁻¹, more preferably, 10⁻⁵ s⁻¹, and most preferably, 10⁻⁶ s⁻¹;
e) the ability to opsonize a cell expressing PSMA; or
f) the ability to inhibit growth and/or mediate phagocytosis and killing of cells expressing PSMA (e.g., a tumor cell) in the presence of human effector cells at a concentration of about 10 µg/ml or less (e.g., *in vitro*).

Examples of tumor cells which can be targeted by the human antibodies of the invention include, but are not limited to, prostate, renal, colon tumor cells.

In one embodiment, isolated human antibodies of the invention bind to PSMA antigen with an affinity constant of at least about 10⁷ M⁻¹, preferably about 10⁸ M⁻¹, more preferably, about 10⁹ M⁻¹, and more preferably about 10¹⁰ to 10¹¹ M⁻¹ or stronger, and are capable of inhibiting growth and/or mediating phagocytosis and killing of cells expressing PSMA by human effector cells, e.g., polymorphonuclear cells (PMNs), monocytes and macrophages, with an IC₅₀ of about 1 x 10⁻⁷ M or less, or at a concentration of about 10 µg/ml or less *in vitro.*

In another aspect, the invention provides nucleic acid molecules encoding the antibodies, or antigen-binding portions, of the invention. Accordingly, recombinant expression vectors which include the antibody-encoding nucleic acids of the invention, and host cells transfected with such vectors, are also encompassed by the invention, as are methods of making the antibodies of the invention by culturing these host cells.

In yet another aspect, the invention provides isolated B-cells from a transgenic non-human animal, e.g., a transgenic mouse, which are capable of expressing various isotypes (e.g., IgG, IgA and/or IgM) of human monoclonal antibodies that specifically bind to PSMA. Preferably, the isolated B cells are obtained from a transgenic non-human animal, e.g., a transgenic mouse, which has been immunized with a purified or enriched preparation of PSMA antigen and/or cells expressing the PSMA. Preferably, the transgenic non-human animal, e.g., a transgenic mouse, has a genome comprising a human heavy chain transgene and a human light chain transgene. The isolated B-cells are then immortalized to provide a source (e.g., a hybridoma) of human monoclonal antibodies to PSMA.

Accordingly, the present invention also provides a hybridoma capable of producing human monoclonal antibodies that specifically bind to PSMA. In one embodiment, the hybridoma includes a B cell obtained from a transgenic non-human animal, e.g., a transgenic mouse, having a genome comprising a human heavy chain transgene and a human light chain transgene fused to an immortalized cell. The transgenic non-human animal can be immunized with a purified or enriched preparation of PSMA antigen and/or cells expressing PSMA to generate antibody-producing hybridomas. Particular hybridomas of the invention include 4A3, 7F12, 8A11, 16F9 and 8C 12, deposited with the ATCC at Accession Numbers ).

In yet another aspect, the invention provides a transgenic non-human animal, such as a transgenic mouse (also referred to herein as a "HuMab"), which express human monoclonal antibodies that specifically bind to PSMA. In a particular embodiment, the transgenic non-human animal is a transgenic mouse having a genome comprising a human heavy chain transgene and a human light chain transgene. The transgenic non-human animal can be immunized with a purified or enriched preparation of PSMA antigen and/or cells expressing PSMA. Preferably, the transgenic non-human animal, e.g., the transgenic mouse, is capable of producing multiple isotypes of human monoclonal antibodies to PSMA (e.g., IgG, IgA and/or IgM) by undergoing V-D-J recombination and isotype switching. Isotype switching may occur by, e.g., classical or non-classical isotype switching.

In another aspect, the present invention provides methods for producing human monoclonal antibodies which specifically react with PSMA. In one embodiment, the method includes immunizing a transgenic non-human animal, e.g., a transgenic mouse, having a genome comprising a human heavy chain transgene and a human light chain transgene, with a purified or enriched preparation of PSMA antigen and/or cells expressing PSMA. B cells (e.g., splenic B cells) of the animal are then obtained and fused with myeloma cells to form immortal, hybridoma cells that secrete human monoclonal antibodies against PSMA.

Isolated anti-PSMA human monoclonal antibodies of the invention, or antigen binding portions thereof, can be derivatized or Linked to another functional molecule, e.g., another peptide or protein (e.g., an Fab' fragment). For example, an antibody or antigen-binding portion of the invention can be functionally linked (e.g., by chemical coupling, genetic fusion, noncovalent association or otherwise) to one or more other molecular entities, such as another antibody (e.g., a bispecific or a multispecific antibody). Accordingly, in another aspect, the present invention features a bispecific or multispecific molecule comprising at least one first binding specificity for PSMA and a second binding specificity an Fc receptor, e.g., human FcγRI or a human Fcα receptor.

Multispecific molecules of the invention also include trispecific, tetraspecific and other multispecific molecules. In one embodiment the multispecific molecule includes an anti-enhancement factor (EF) portion, e.g., a molecule which binds to a surface protein involved in cytotoxic activity.

In a particular embodiment, bispecific and multispecific molecules of the invention comprise at least one antibody, or fragment thereof (e.g., an Fab, Fab', F(ab')₂, Fv, or a single chain Fv). In a particular embodiment, the antibody or fragment thereof is a completely human antibody or a portion thereof, or a "chimeric" or a "humanized" antibody or a portion thereof (e.g., has a variable region, or at least a complementarity determining region (CDR), derived from a non-human antibody (e.g., murine) with the remaining portion(s) being human in origin).

In one embodiment, the at least one antibody or fragment thereof of the bispecific or multispecific molecule binds to an Fe receptor, such as a human IgG receptor, e.g., an Fc-gamma receptor (FcγR), such as FcγRI (CD64), FcγRII(CD32), and FcγRIII (CD16). A preferred Fcγ receptor is the high affinity Fcγ receptor, FcγRI. However, other Fc receptors, such as human IgA receptors (e.g. FcαRI) also can be targeted. The Fc receptor is preferably located on the surface of an effector cell, e.g., a monocyte, macrophage or an activated polymorphonuclear cell. In a preferred embodiment, the bispecific and multispecific molecules bind to an Fc receptor at a site which is distinct from the immunoglobulin Fc (e.g., IgG or IgA) binding site of the receptor. Therefore, the binding of the bispecific and multispecific molecules is not blocked by physiological levels of immunoglobulins.

In another aspect, the present invention features a human anti-PSMA antibody, or a fragment thereof, conjugated to a therapeutic moiety, e.g., a cytotoxic drug, an enzymatically active toxin, or a fragment thereof, a radioisotope, or a small molecule anti-cancer drug.

In another aspect, the present invention provides target-specific effector cells which comprise an effector cell expressing an Fc receptor, e.g., a macrophage or an activated PMN cell, and a bispecific or multispecific molecule of the invention.

In another aspect, the present invention provides compositions, e.g., pharmaceutical and diagnostic compositions, comprising a pharmaceutically acceptable carrier and at least one human monoclonal antibody of the invention, or an antigen-binding portion thereof, which specifically binds to PSMA. In one embodiment, the composition comprises a combination of the human antibodies or antigen-binding portions thereof, preferably each of which binds to a distinct epitope. For example, a pharmaceutical composition comprising a human monoclonal antibody that mediates highly effective killing of target cells in the presence of effector cells can be combined with another human monoclonal antibody that inhibits the growth of cells expressing PSMA. Thus, the combination provides multiple therapies tailored to provide the maximum therapeutic benefit. Compositions, e.g., pharmaceutical compositions, comprising a combination of at least one human monoclonal antibody of the invention, or antigen-binding portions thereof, and at least one bispecific or multispecific molecule of the invention, are also within the scope of the invention.

In yet another aspect, the invention provides a method for inhibiting the proliferation and/or differentiation of a cell expressing PSMA by inhibiting growth and/or by inducing phagocytosis and/or killing of target cells by human effector cells, such as human polymorphonuclear cells (PMNs), monocytes and macrophages, using an antibody, or antigen-binding portion thereof (or a bispecific or multispecific antibody) of the invention. In one embodiment, the method comprises contacting a cell expressing PSMA either *in vitro* or *in vivo* with one or a combination of human monoclonal antibodies of the invention, or an antigen-bindiug portion thereof, in the presence of a human effector cell. The method can be employed in culture, e.g. *in vitro* or *ex vivo* (e.g., cultures comprising cells expressing PSMA and effector cells). For example, a sample containing cells expressing PSMA and effector cells can be cultured *in vitro,* and combined with an antibody of the invention, or an antigen-binding portion thereof (or a bispecific or multispecific molecule of the invention). Alternatively, the method can be performed in a subject, e.g., as part of an *in vivo* (e.g., therapeutic or prophylactic) protocol.

For in vivo methods, the antibody, or antigen-binding portion thereof (or a bispecific or multispecific molecule of the invention), can be administered to a human subject suffering from a PSMA-related disease, such as cancers of the prostate, kidney and colon, such that growth inhibition, phagocytosis and/or killing of cells expressing PSMA is induced. In one embodiment, the subject can be additionally treated with an agent that modulates, e.g., enhances or inhibits, the expression or activity of Fc receptor, e.g., an Fcα receptor or an Fcγ receptor, by for example, treating the subject with a cytokine. Preferred cytokines for administration during treatment with the bispecific and multispecific molecule include granulocyte colony-stimulating factor (G-CSF), granulocyte- macrophage colony-stimulating factor (GM-CSF), interferon-γ (IFN-γ), and tumor necrosis factor (TNF).

Isolated human monoclonal antibody compositions of the invention also can be administered in combination with other known therapies, e.g., an anti-cancer therapy.

Exemplary diseases that can be treated (e.g., ameliorated) or prevented using the methods and compositions of the invention include, but are not limited to, tumorigenic diseases. Such tumorigenic diseases include cancers of the prostate, kidney and colon.

In yet another aspect, the present invention provides a method for detecting in *vitro* or in vivo the presence of PSMA antigen in a sample, e.g., for diagnosing a PSMA-related disease. In one embodiment, this is achieved by contacting a sample to be tested, along with a control sample, with a human monoclonal antibody of the invention, or an antigen-binding portion thereof (or a bispecific or multispecific molecule), under conditions that allow for formation of a complex between the antibody and PSMA. Complex formation is then detected (e.g., using an ELISA) in both samples, and any statistically significant difference in the formation of complexes between the samples is indicative the presence of PSMA antigen in the test sample.

Other features and advantages of the instant invention be apparent from the following detailed description and claims.

### Brief Description of the Drawings

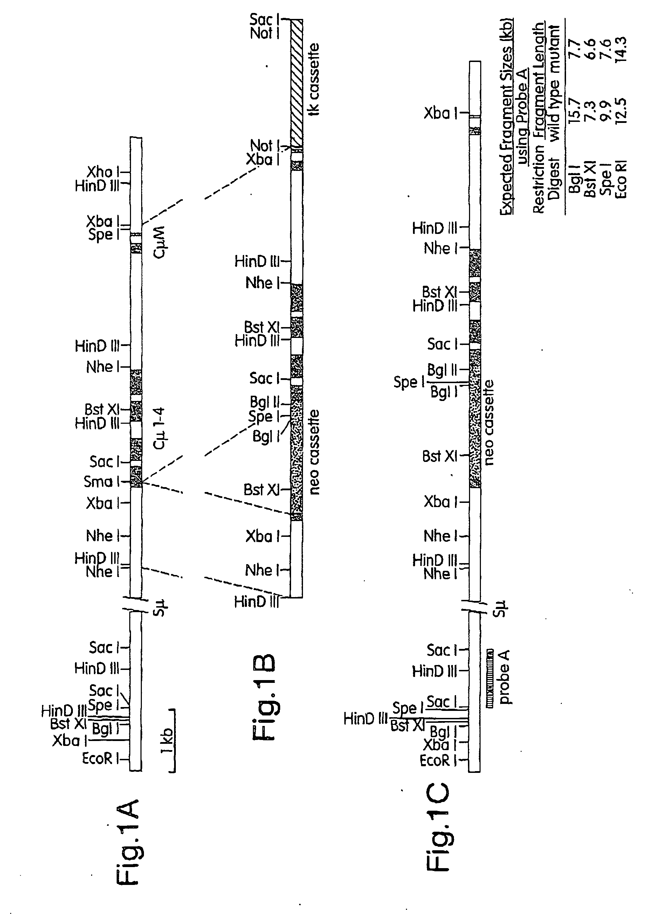

*Figure 1* is a schematic diagram of the targeted insertion of a neo cassette into the SmaI site of the µl exon. *Panel A* depicts the genomic structure of the µ locus, where the filled boxes represent the µ exons. *Panel B* is a schematic diagram of the CmD targeting vector. *Panel C* depicts the targeted µ locus in which the neo cassette has been inserted into µl.

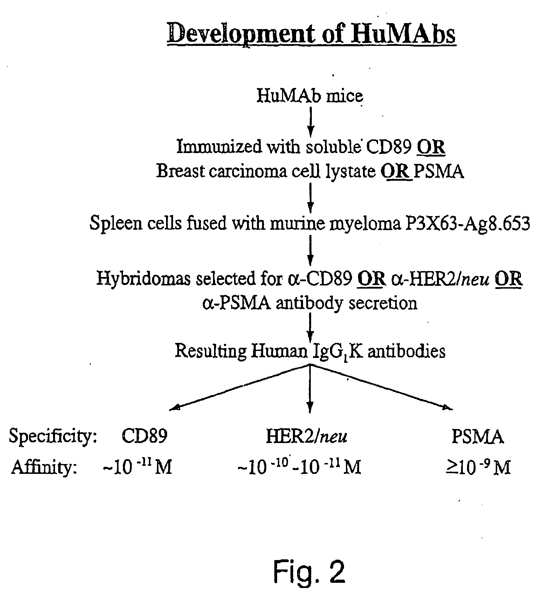

*Figure 2* is a schematic description of the production of IgG₁K human monoclonal antibodies (HuMabs) against PSMA, HER2/*neu* and CD89 (FcαR) by immunizing transgenic mice.

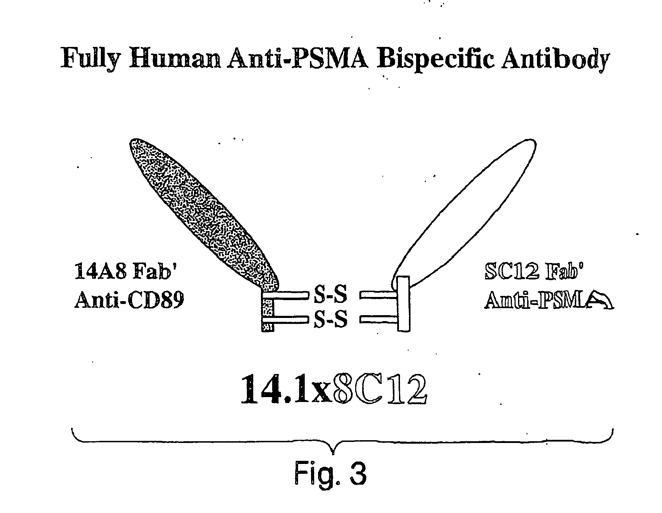

*Figure 3* shows a fully human bispecific molecule, 14.1 x 8C12, which binds to CD89 (FcaR) and to PSMA. The molecule contains an anti-CD89 F ab' antibody fragment (derived from human monoclonal auti-CD89 antibody,14.1) chemically linked by disulfide bond to an anti-PSMA F ab' antibody fragment (derived from human monoclonal anti-PSMA antibody, 8C12).

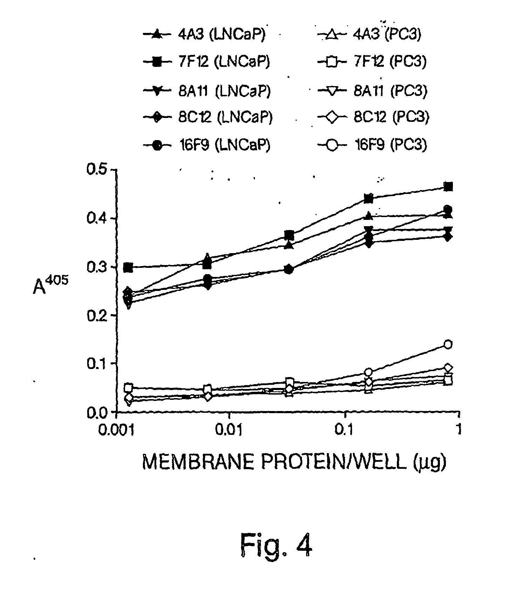

*Figure 4* shows the reactivity of human anti-PSMA monoclonal antibodies with membrane fractions from human prostatic adenocarcinoma LNCaP and PC3 cells as assessed by ELISA. Background absorbance at 405 nm was 0.05.

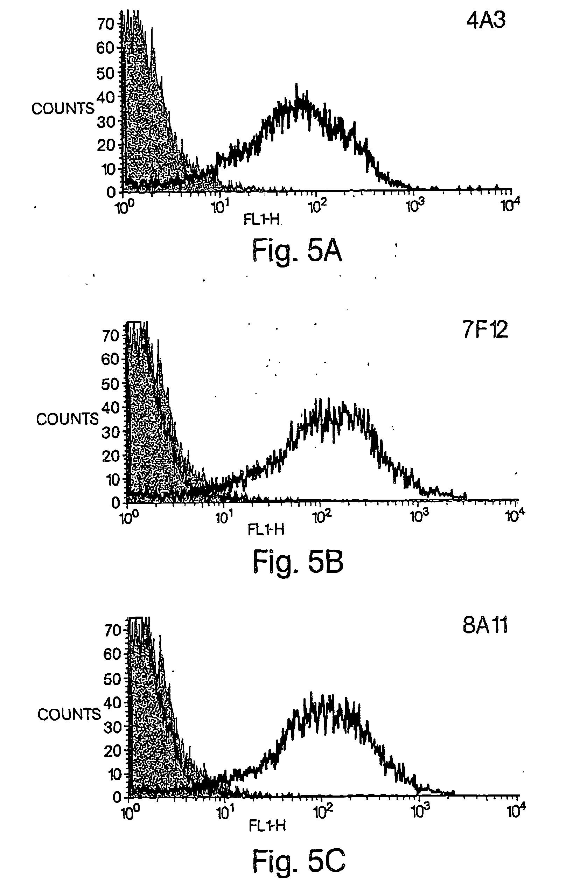

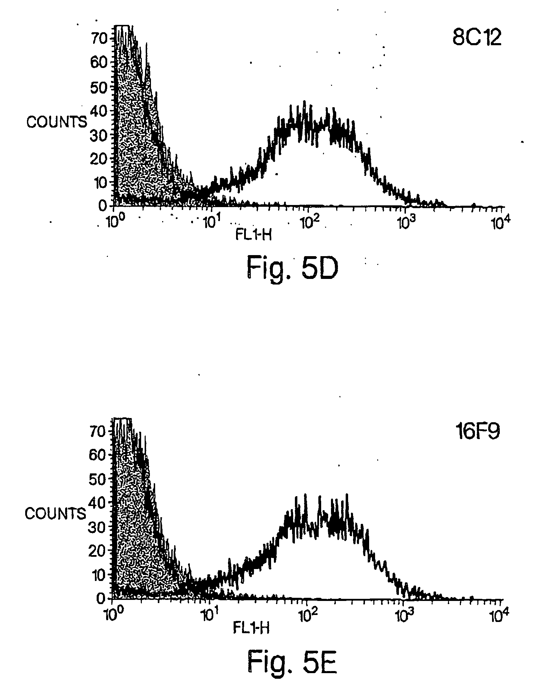

*Figure 5* shows the binding of human anti-PSMA antibodies to LNCaP and PC3 tumor cells as assessed by flow cytometric analysis. Green histogram- binding to LNCaP cells; Pink histogram- binding to PC3 cells; Purple histogram- irrelevant human IgGl. Panel A, antibody 4A3; Panel B, antibody 7F12; Panel C, antibody 8A11; Panel D, antibody 8C12; Panel E, antibody 16F9.

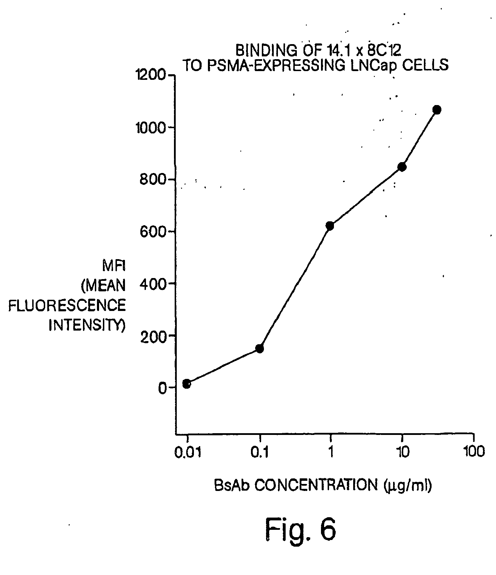

*Figure 6* is a graph showing binding the 14.1 x 8C12 bispecific molecule shown in Figure 3 to PSMA-expressing LNCaP cells as a function bispecific molecule concentration. Binding was measured by mean fluorescence intensity.

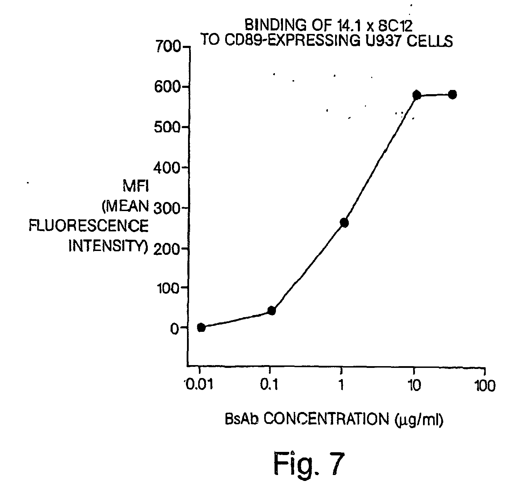

*Figure 7* is a graph showing binding of the 14.1 x 8C12 bispecific molecule shown in Figure 3 to CD89-expressing U937 cells as a function of bispecific molecule concentration. Binding was measured by mean fluorescence intensity.

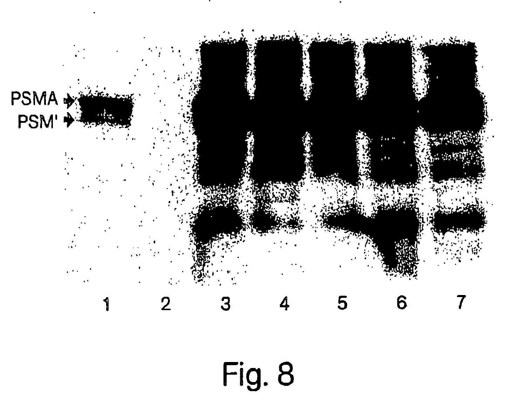

*Figure 8* shows immunoprecipitation of PSMA from LNCaP cell detergent lysates using human anti-PSMA specific antibodies. Lane 1, LNCaP cell lysate; lanes 2 through 7, immunoprecipitation with the following antibodies: irrelevant human IgG1, 4A3, 7F12, 8A11, 8C12 and 16F9, respectively. Immune complexes were analyzed by SDS gel electrophoresis and Western blotting using the murine anti-PSMA antibody 4D8.

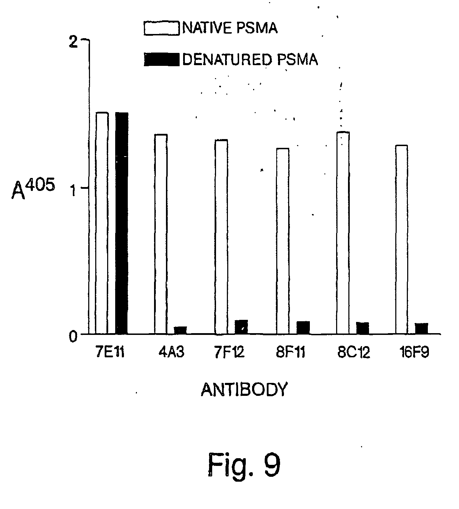

*Figure 9* demonstrates the effect of heat denaturation of isolated PSMA on human anti-PSMA antibody binding.

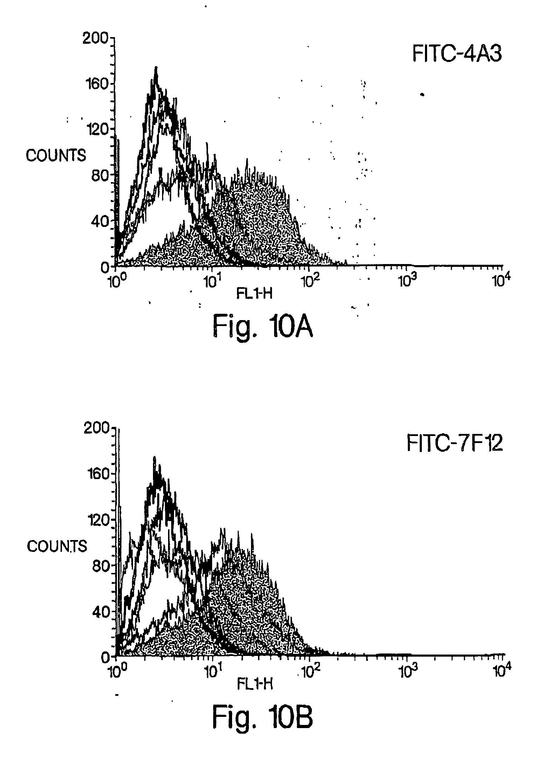

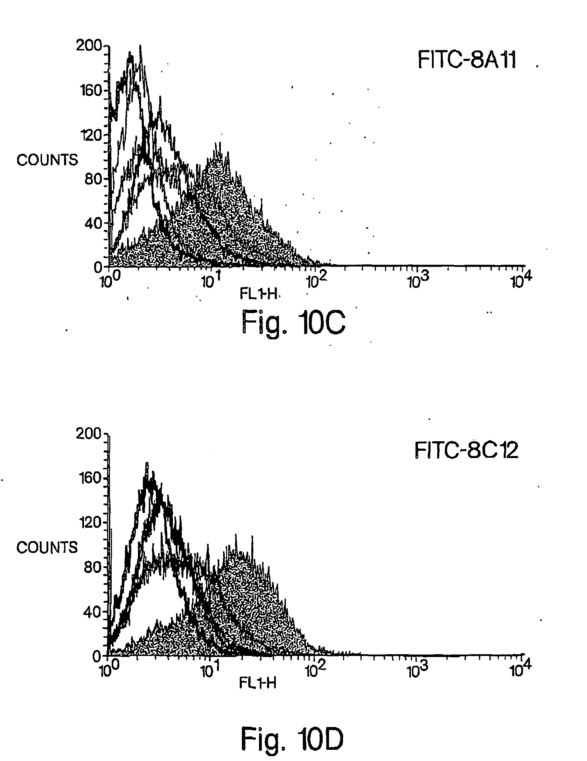

*Figure 10* shows a competitive binding analysis of the human anti-PSMA antibodies, as measured by flow cytometry. Pretreatment was conducted with the following antibodies: Irrelevant human IgGl (solid purple histogram); 4A3 (yellow histogram); 7F12 (green histogram); 8A11 (blue histogram); 8C12 (pink histogram); and 16F9 (orange histogram). Panel A, FITC A43; Panel B, FITC 7F12; Panel C, FITC 8A11; and Panel D, FITC 8C12.

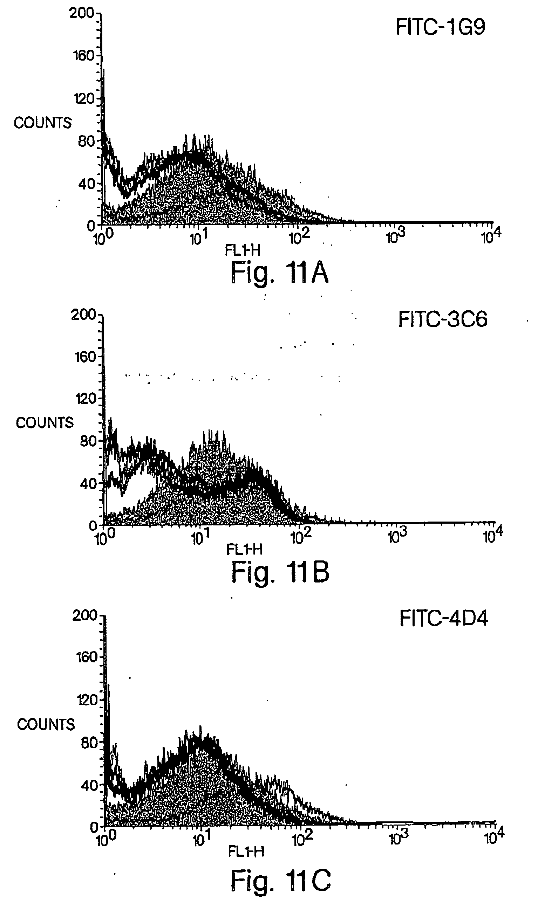

*Figure 11* shows a competitive binding analysis of human anti-PSMA antibodies versus murine anti-PSMA conformational antibodies, as measured by flow cytometry. Pretreatment was conducted with the following antibodies: Irrelevant human IgGl (solid purple histogram); 4A3 (yellow histogram); 7F12 (green histogram); 8A11 (blue histogram); 8C12 (pink histogram); and 16F9 (orange histogram). Panel A, FITC IG9; Panel B, FITC 3C6; and Panel C, FITC 4D4.

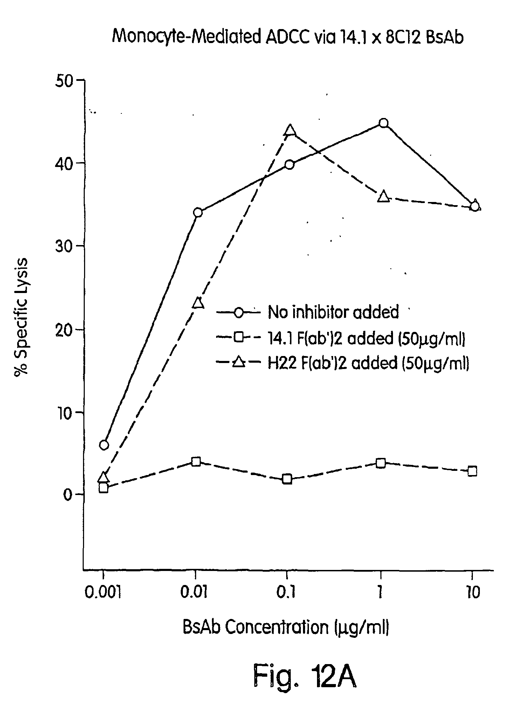

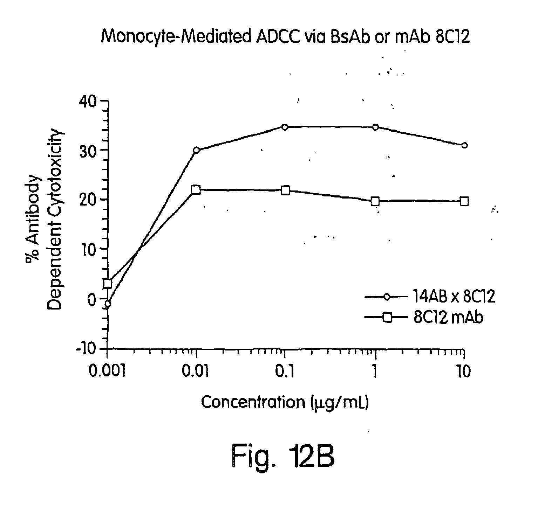

*Figure 12, Panel A* is a graph showing monocyte-mediated antibody dependent cell cytotoxicity (ADCC) of PSMA-expressing cells via the 14.1 x 8C12 bispecific molecule shown in Figure 3 as a function bispecific molecule concentration. Results were measured as a percent of specific cell lysis using no added inhibitor, 50 µg/ml free anti-FcRαR (14.1) F(ab')2 and 50 µg/ml free anti-FcγRI (H22) F(ab')2; *Panel B* is a graph showing monocyte-mediated antibody dependent cell cytotoxicity (ADCC) of LNCaP cells via the bispecific molecule 14A8 x 8C12 and monoclonal antibody 8C12 at an effector:target ratio of 100:1; *Panel C* is a graph showing monocyte-mediated antibody dependent cell cytotoxicity (ADCC) of LNCaP cells via the 14A8 x 8C12 bispecific molecule in the absence of inhibitor, or in the presence of excess amouts of 14A8 F(ab')2 or H22 F(ab')2, and at an effector:target ratio of 100:1.

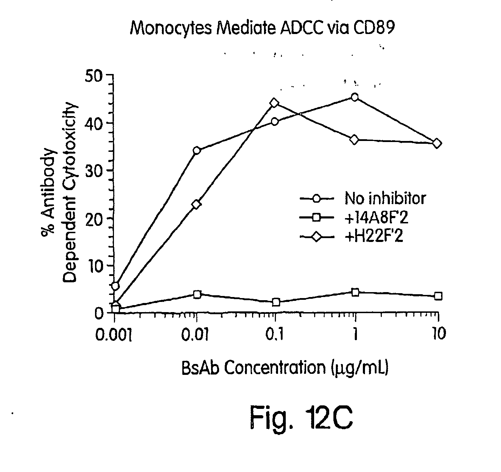

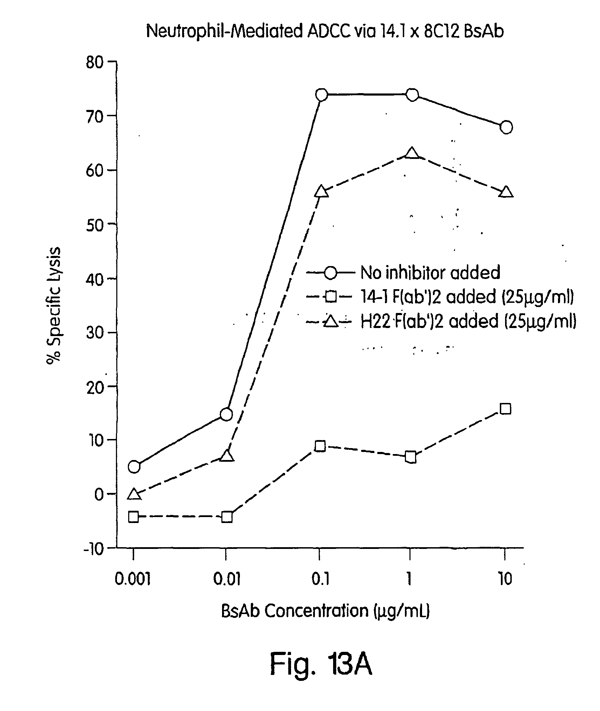

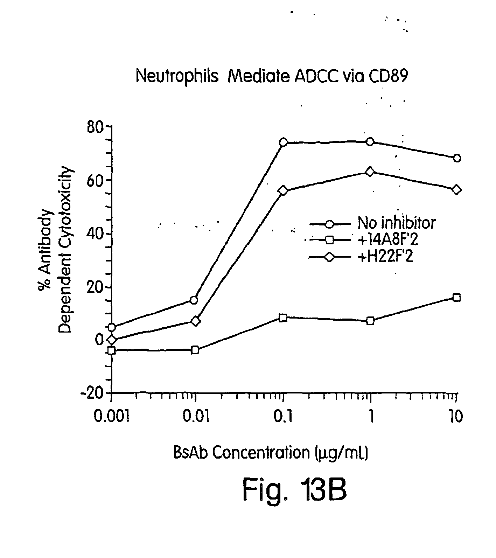

*Figure 13, Panel A* is a graph showing neutrophil-mediated antibody dependent cell cytotoxicity (ADCC) of PSMA-expressing cells via the 14.1 x 8C12 bispecific molecule shown in Figure 3 as a function bispeeific molecule concentration. Results were measured as a percent of specific cell lysis using no added inhibitor, 25 µg/ml free anti-FcRαR (14.1) F(ab')2 and 25 µg/ml free anti-FcγRI (H22) F(ab')2; *Panel B* is a graph showing neutrophil-mediated antibody dependent cell cytotoxicity (ADCC) of LNCaP cells via the 14A8 x 8C12 bispecific molecule in the absence of inhibitor, or in the presence of excess amouts of 14A8 F(ab')2 or H22 F(ab')2, and at an effector:target ratio of 200:1.

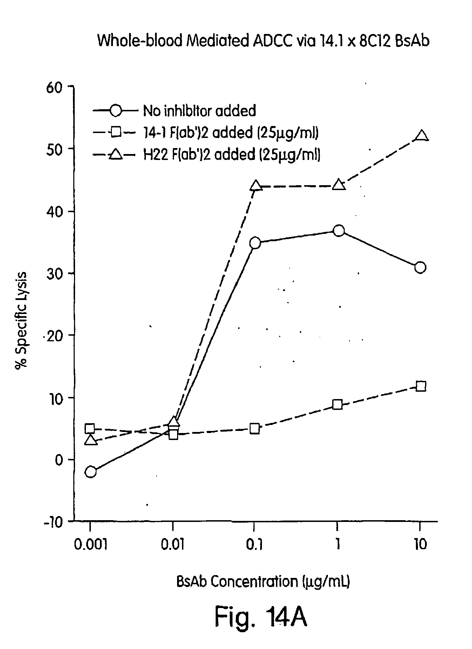

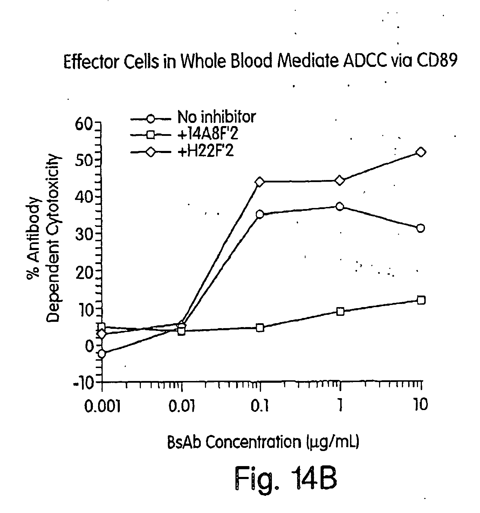

*Figure 14, Panel A* is a graph showing whole blood-mediated antibody dependent cell cytotoxicity (ADCC) of PSMA-expressing cells via the 14.1 x 8C12 bispecific molecule shown in Figure 3 as a function bispecific molecule concentration. Results were measured as a percent of specific cell lysis using no added inhibitor, 25 µg/ml free anti-FcRaR (14.1) F(ab')2 and 25 µg/ml free anti-FcγRI (H22) F(ab')2; *Panel B* is a graph showing whole blood-mediated antibody dependent cell cytotoxicity (ADCC) of LNCaP cells via the 14A8 x 8C12 bispecific molecule in the absence of inhibitor, or in the presence of excess amouts of 14A8 F(ab')2 or H22 F(ab')2.

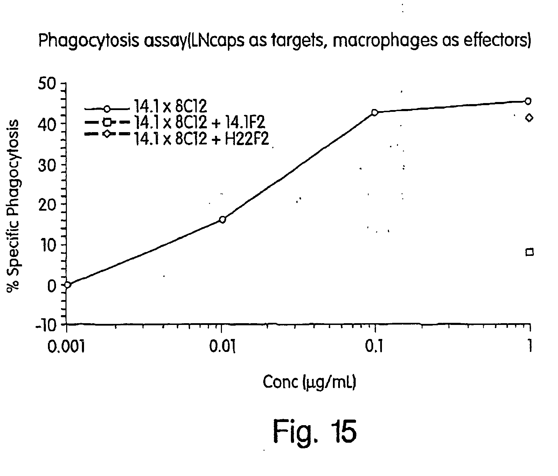

*Figure 15* is a graph showing bispecific molecule (14.1 x 8C12) -mediated phagocytosis of PSMA expressing (LnCAP) cells by monocyte derived macrophages (MDM) (circles). Results were measured as a percent of phagocytosis both in the presence and absence of excess 14.1 antibody as an inhibitor (squares) and H22 antibody as a control (diamonds).

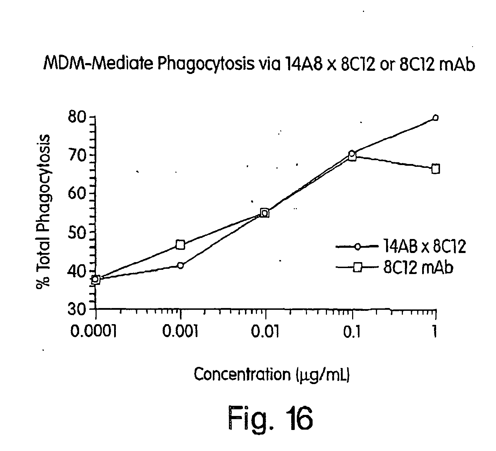

*Figure 16* is a graph showing bispecific molecule (14A8 x 8C12)-mediated phagocytosis and antibody (8C12)- mediated phagocytosis of LnCAP tumor cells by monocyte derived macrophages (MDM).

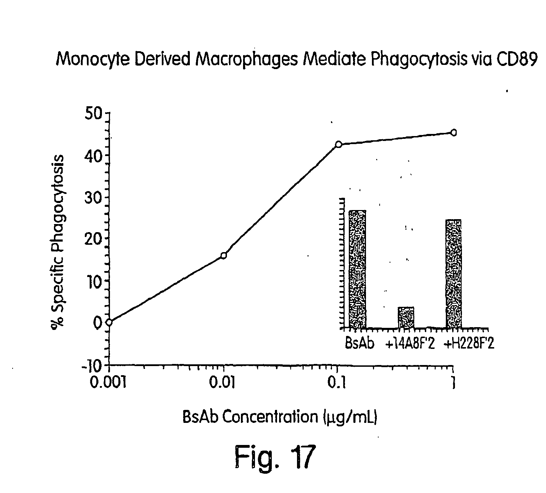

*Figure 17* is a graph showing bispecific molecule (14A8 x 8C12)-mediated phagocytosis of LnCAP tumor cells by monocyte derived macrophages (MDM) (circles). The inset is a graph showing phagocytosis mediated by the 14A8 x 8C12 bispecific molecule (1 µg/ml) in the presence of excess 14A8 F(ab')2 or H22 F(ab')2.

### Detailed Description of the Invention

The present invention provides novel antibody-based therapies for treating and diagnosing diseases characterized by expression, particularly over-expression, of prostate-specific membrane antigen (referred to herein as "PSMA") and/or related molecules. Therapies of the invention employ isolated human monoclonal antibodies, or antigen-binding portions thereof, which bind to an epitope present on PSMA. In one embodiment, the human antibodies are produced in a non-human transgenic animal, e.g., a transgenic mouse, capable of producing multiple isotypes of human monoclonal antibodies to PSMA (e.g., IgG, IgA and/or IgE) by undergoing V-D-J recombination and isotype switching. Accordingly, various aspects of the invention include antibodies and antibody fragments, and pharmaceutical compositions thereof, as well as non-human transgenic animals, and B-cells and hybridomas for making such monoclonal antibodies. Methods of using the antibodies of the invention to detect a cell expressing PSMA or a related, cross-reactive growth factor receptor, or to inhibit growth, differerttiation and/or motility of a cell expressing PSMA, either *in vitro* or *in* vivo, are also encompassed by the invention.

In order that the present invention may be more readily understood, certain terms are first defined. Additional definitions are set forth throughout the detailed description.

The terms "prostate-specific membrane antigen," "PSMA" and ''PSMA antigen" are used interchangeably herein, and include variants, isoforms and species homologs of human PSMA. In a preferred embodiment, binding of an antibody of the invention to the PSMA-antigen inhibits the growth of cells expressing PSMA (e.g., a tumor cell). In another preferred embodiment, binding of an antibody of the invention to the PSMA-antigen mediates effector cell phagocytosis and/or killing of cells expressing PSMA.

As used herein, the term "antibody" refers to a glycoprotein comprising at least two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "antigen-binding portion" of an antibody (or simply "antibody portion"), as used herein, refers to one or more fragments of an antibody that retain the ability to specifically bind to an antigen (*e.g*., PSMA). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

The term "bispecific molecule" is intended to include any agent, e.g., a protein, peptide, or protein or peptide complex, which has two different binding specificities which bind to, or interact with (a) a cell surface antigen and (b) an Fc receptor on the surface of an effector cell. The term "multispecific molecule" or "heterospecific molecule" is intended to include any agent, e.g., a protein, peptide, or protein or peptide complex, which has more than two different binding specificities which bind to, or interact with (a) a cell surface antigen, (b) an Fc receptor on the surface of an effector cell, and (c) at least one other component. Accordingly, the invention includes, but is not limited to, bispecific, trispecific, tetraspecific, and other multispecific molecules which are directed to cell surface antigens, such as PSMA, and to Pc receptors on effector cells. The term "bispecific antibodies" further includes diabodies. Diabodies are bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad Sci. USA 90:6444-6448; Poljak, RJ., et al. (1994) Structure 2:1121-1123).

As used herein, the term "heteroantibodies" refers to two or more antibodies, antibody binding fragments (e.g., Fab), derivatives therefrom, or antigen binding regions linked together, at least two of which have different specificities. These different specificities include a binding specificity for an Fc receptor on an effector cell, and a binding specificity for an antigen or epitope on a target cell, e.g., a tumor cell. The term "human antibody", as used herein, is intended to include antibodies having variable and constant regions derived from human germline immunoglobulin sequences. The human antibodies of the invention may include amino acid residues not encoded by human germline immunoglobulin sequences (*e.g.*, mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences.

The terms "monoclonal antibody" or "monoclonal antibody composition" as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g., a transgenic mouse, having a genome comprising a human heavy chain transgene and a light chain transgene fused to an immortalized cell.

The term "recombinant human antibody", as used herein, is intended to include all human antibodies that are prepared, expressed, created or isolated by recombinant means, such as antibodies isolated from an animal (e.g., a mouse) that is transgenic for human imznunoglobulin genes (described further in Section I, below); antibodies expressed using a recombinant expression vector transfected into a host cell, antibodies isolated from a recombinant, combinatorial human antibody library, or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

As used herein, a "heterologous antibody" is defined in relation to the transgenic non-human organism producing such an antibody. This term refers to an antibody having an amino acid sequence or an encoding nucleic acid sequence corresponding to that found in an organism not consisting of the transgenic non-human animal, and generally from a species other than that of the transgenic non-human animal.

As used herein, a "heterohybrid antibody" refers to an antibody having a light and heavy chains of different organismal origins. For example, an antibody having a human heavy chain associated with a murine light chain is a heterohybrid antibody. Examples of heterohybrid antibodies include chimeric and humanized antibodies, discussed supra.

An "isolated antibody", as used herein, is intended to refer to an antibody which is substantially free of other antibodies having different antigenic specificities (*e.g*., an isolated antibody that specifically binds to PSMA is substantially free of antibodies that specifically bind antigens other than PSMA). An isolated antibody that specifically binds to an epitope, isoform or variant of human PSMA may, however, have cross-reactivity to other related antigens, e.g., from other species (e.g., PSMA species homologs). Moreover, an isolated antibody may be substantially free of other cellular material and/or chemicals. In one embodiment of the invention, a combination of "isolated" monoclonal antibodies having different specificities are combined in a well defined composition.

As used herein, "specific binding" refers to antibody binding to a predetermined antigen. Typically, the antibody binds with an affinity of at least about 1 x 10⁷ M⁻¹, and binds to the predetermined antigen with an affinity that is at least two-fold greater than its affinity for binding to a non-specific antigen (e.g., BSA, casein) other than the predetermined antigen or a closely-related antigen. The phrases "an antibody recognizing an antigen" and "an antibody specific for an antigen" are used interchangeably herein with the term "an antibody which binds specifically to an antigen".

As used herein, the term "high affinity" for an IgG antibody refers to a binding affinity of at least about 10⁷M⁻¹, preferably at least about 10⁹M⁻¹, more preferably at least about 10¹⁰M⁻¹,10¹¹M⁻¹, 10¹²M⁻¹ or greater, e.g., up to 10¹³M⁻¹ or greater. However, "high affinity" binding can vary for other antibody isotypes. For example, "high affinity" binding for an IgM isotype refers to a binding affinity of at least about 1 x 10⁷M⁻¹.

The term "K_{assoc}", as used herein, is intended to refer to the association constant of a particular antibody-antigen interaction.

The term "K_{dis}", as used herein, is intended to refer to the dissociation constant of a particular antibody-antigen interaction.

As used herein, "isotype" refers to the antibody class (e.g., IgM or IgGl) that is encoded by heavy chain constant region genes.

As used herein, "isotype switching" refers to the phenomenon by which the class, or isotype, of an antibody changes from one Ig class to one of the other Ig classes.

As used herein, "nonswitched isotype" refers to the isotypic class of heavy chain that is produced when no isotype switching has taken place; the CH gene encoding the nonswitched isotype is typically the first CH gene immediately downstream from the functionally rearranged VDJ gene. Isotype switching has been classified as classical or non-classical isotype switching. Classical isotype switching occurs by recombination events which involve at least one switch sequence region in the transgene. Non-classical isotype switching may occur by, for example, homologous recombination between human σ_{µ} and human Σ_{µ} (δ-associated deletion). Alternative non-classical switching mechanisms, such as intertransgene and/or interchromosomal recombination, among others, may occur and effectuate isotype switching.

As used herein, the term "switch sequence" refers to those DNA sequences responsible for switch recombination. A "switch donor" sequence, typically a µ switch region, will be 5' (i.e., upstream) of the construct region to be deleted during the switch recombination. The "switch acceptor" region will be between the construct region to be deleted and the replacement constant region (e.g., γ, ε, etc.). As there is no specific site where recombination always occurs, the final gene sequence will typically not be predictable from the construct.

As used herein, "glycosylation pattern" is defined as the pattern of carbohydrate units that are covalently attached to a protein, more specifically to an immunoglobulin protein. A glycosylation pattern of a heterologous antibody can be characterized as being substantially similar to glycosylation patterns which occur naturally on antibodies produced by the species of the nonhuman transgenic animal, when one of ordinary skill in the art would recognize the glycosylation pattern of the heterologous antibody as being more similar to said pattern of glycosylation in the species of the nonhuman transgenic animal than to the species from which the CH genes of the transgene were derived.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring.

The term "rearranged" as used herein refers to a configuration of a heavy chain or light chain immunoglobulin locus wherein a V segment is positioned immediately adjacent to a D-J or J segment in a conformation encoding essentially a complete VH or VL domain, respectively. A rearranged immunoglobulin gene locus can be identified by comparison to germline DNA; a rearranged locus will have at least one recombined heptamer/nonamer homology element.

The term "unrearranged" or "germline configuration" as used herein in reference to a V segment refers to the configuration wherein the V segment is not recombined so as to be immediately adjacent to a D or J segment.

The term "nucleic acid molecule", as used herein, is intended to include DNA molecules and RNA molecules. A nucleic acid molecule may be single-stranded or double-stranded, but preferably is double-stranded DNA.

The term "isolated nucleic acid molecule", as used herein in reference to nucleic acids encoding antibodies or antibody portions (*e.g*., VH, VL, CDR3) that bind to PSMA, is intended to refer to a nucleic acid molecule in which the nucleotide sequences encoding the antibody or antibody portion are free of other nucleotide sequences encoding antibodies or antibody portions that bind antigens other than PSMA, which other sequences may naturally flank the nucleic acid in human genomic DNA. SEQ ID NOS: 1-12 comprise the nucleotide and amino acid sequences comprising the heavy chain (VH) and light chain (VL) variable regions of the 3.F2, 1.D2 and 2.E8 human anti-PSMA monoclonal antibodies of the invention.

For nucleic acids, the term "substantial homology" indicates that two nucleic acids, or designated sequences thereof, when optimally aligned and compared, are identical, with appropriate nucleotide insertions or deletions, in at least about 80% of the nucleotides, usually at least about 90% to 95%, and more preferably at least about 98% to 99.5% of the nucleotides. Alternatively, substantial homology exists when the segments will hybridize under selective hybridization conditions, to the complement of the strand.

The percent identity between two sequences is a function of the number of identical positions shared by the sequences (i.e., % homology = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two nucleotide sequences can be determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. The percent identity between two nucleotide or amino acid sequences can also determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol, (48):444-453 (1970)) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The nucleic acid and protein sequences of the present invention can further be used as a "query sequence" to perform a search against public databases to, for example, identify related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength =12 to obtain nucleotide sequences homologous to the nucleic acid molecules of the invention. BLAST protein searches can be performed with the XBLAST program, score = 50, wordlength = 3 to obtain amino acid sequences homologous to the protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used. See http://www.ncbi.nlm.nih.gov.

The nucleic acids may be present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art. *See,* F. Ausubel, et al., ed. Current Protocols in Molecular Biology, Greene Publishing and Wiley Interscience, New York (1987).

The nucleic acid compositions of the present invention, while often in a native sequence (except for modified restriction sites and the like), from either cDNA, genomic or mixtures may be mutated, thereof in accordance with standard techniques to provide gene sequences. For coding sequences, these mutations, may affect amino acid sequence as desired. In particular, DNA sequences substantially homologous to or derived from native V, D, J, constant, switches and other such sequences described herein are contemplated (where "derived" indicates that a sequence is identical or modified from another sequence).

A nucleic acid is "operably linked" when it is placed into a functional relationship with another nucleic acid sequence. For instance, a promoter or enhancer is operably linked to a coding sequence if it affects the transcription of the sequence. With respect to transcription regulatory sequences, operably linked means that the DNA sequences being linked are contiguous and, where necessary to join two protein coding regions, contiguous and in reading frame. For switch sequences, operably linked indicates that the sequences are capable of effecting switch recombination.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g*., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g.,* replication defective retroviruses, adenoviruses and adeno-associated viruses), which serve equivalent functions.

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

Various aspects of the invention are described in further detail in the following subsections.

### I. Production of Human Antibodies to PSMA

The monoclonal antibodies (mAbs) of the invention can be produced by a variety of techniques, including conventional monoclonal antibody methodology e.g., the standard somatic cell hybridization technique of Kohler and Milstein, Nature 256: 495 (1975). Although somatic cell hybridization procedures are preferred, in principle, other techniques for producing monoclonal antibody can be employed e.g., viral or oncogenic transformation of B lymphocytes.

The preferred animal system for preparing hybridomas is the murine system. Hybridoma production in the mouse is a very well-established procedure. Immunization protocols and techniques for isolation of immunized splenocytes for fusion are known in the art. Fusion partners (e.g., murine myeloma cells) and fusion procedures are also known.

In a preferred embodiment, human monoclonal antibodies directed against PSMA can be generated using transgenic mice carrying parts of the human immune system rather than the mouse system. These transgenic mice, referred to herein as "HuMAb" mice, contain a human immunoglobulin gene miniloci that encodes unrearranged human heavy (µ and γ) and κ light chain immunoglobulin sequences, together with targeted mutations that inactivate the endogenous µ and κ chain loci (Lonberg, N. et al. (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg, N. *et al.* (1994), *supra;* reviewed in Lonberg, N. (1994) Handbook of Experimental Pharmacology 113:49-101; Lonberg, N. and Huszar, D. (1995) Intern. Rev. Immunol. Vol. 13: 65-93, and Harding. F. and Lonberg, N. (1995) Ann. N.Y. Acad Sci 764:536-546). The preparation of HuMab mice is described in detail Section II below and in Taylor, L. et al. (1992) Nucleic Acids Research 20:6287-6295; Chen, J. et al. (1993) International Immunology 5: 647-656; Tuaillon et al. (1993) Proc. Natl. Acad Sci USA 90:3720-3724; Choi et al. (1993) Nature Genetics 4:117-123; Chen, J. et al. (1993) EMBO J. 12: 821-830; Tuaillon et al. (1994) J. Immunol. 152:2912-2920; Lonberg et al., (1994) Nature 368(6474): 856-859; Lonberg, N. (1994) Handbook of Experimental Pharmacology 113:49-101; Taylor, L. et al. (1994) International Immunology 6: 579-591; Lonberg, N. and Huszar, D. (1995) Intern. Rev. Immunol. Vol. 13:65-93; Harding, F. and Lonberg, N. (1995) Ann. N. Y. Acad. Sci 764:536-546; Fishwild, D. et al. (1996) Nature Biotechnology 14: 845-851, the contents of all of which are hereby incorporated by reference in their entirety. See further, U.S. Patent Nos. 5,545,806; 5,569,825; 5,625,126; 5,633,425; 5,789,650; 5,877,397; 5,661,016; 5,814,318; 5,874,299; and 5,770,429; all to Lonberg and Kay, and GenPharm International; U.S. Patent No. 5,545,807 to Surani *et al.;* International Publication Nos. WO 98/24884, published on June 11, 1998; WO 94/25585, published November 10, 1994; WO 93/1227, published June 24,1993; WO 92/22645, published December 23,1992; WO 92/03918, published March 19,1992, the disclosures of all of which are hereby incorporated by reference in their entity. Alternatively, the HCO12 transgenic mice described in Example 2, can be used to generate human anti-PSMA antibodies.

### HuMab Immunizations

To generate fully human monoclonal antibodies to PSMA, HuMab mice can be immunized with a purified or enriched preparation of PSMA antigen and/or cells expressing PSMA, as described by Lonberg, N. et al. (1994) Nature 368(6474): 856-859; Fishwild, D. et al. (1996) Nature Biotechnology 14: 845-851 and WO 98/24884. Preferably, the mice will be 6-16 weeks of age upon the first infusion. For example, a purified or enriched preparation (5-20 µg) of PSMA antigen (e.g., purified from PSMA-expressing LNCaP cells) can be used to immunize the HuMab mice intraperitoneally. In the event that immunizations using a purified or enriched preparation of PSMA, antigen do not result in antibodies, mice can also be immunized with cells expressing PSMA, e.g., a tumor cell line, to promote immune responses.

Cumulative experience with various antigens has shown that the HuMAb transgenic mice respond best when initially immunized intraperitoneally (IP) with antigen in complete Freund's adjuvant, followed by every other week IP immunizations (up to a total of 6) with antigen in incomplete Freund's adjuvant. The immune response can be monitored over the course of the immunization protocol with plasma samples being obtained by retroorbital bleeds. The plasma can be screened by ELISA (as described below), and mice with sufficient titers of anti-PSMA human immunoglobulin can be used for fusions. Mice can be boosted intravenously with antigen 3 days before sacrifice and removal of the spleen. It is expected that 2-3 fusions for each antigen may need to be performed. Several mice will be immunized for each antigen. For example, a total of twelve HuMAb mice of the HC07 and HC012 strains can be immunized.

### Generation of Hybridomas Producing Human Monoclonal Antibodies to PSMA

The mouse splenocytes can be isolated and fused with PEG to a mouse myeloma cell line based upon standard protocols (8, 13). The resulting hybridomas are then screened for the production of antigen-specific antibodies. For example, single cell suspensions of splenic lymphocytes from immunized mice are fused to one-sixth the number of P3X63-Ag8.653 nonsecreting mouse myeloma cells (ATCC, CRL 1580) with 50% PEG. Cells are plated at approximately 2 x 10⁵ in flat bottom microtiter plate, followed by a two week incubation in selective medium containing 20% fetal Clone Serum, 18% "653" conditioned media, 5% origen (IGEN), 4 mM L-glutamine, 1 mM L~glutamine, 1 mM sodium pyruvate, 5mM HEPES, 0.055 mM 2-mercaptoethanol, 50 units/ml penicillin, 50 mg/ml streptomycin, 50 mg/ml gentamycin and 1X HAT (Sigma; the HAT is added 24 hours after the fusion). After two weeks, cells are cultured in medium in which the HAT is replaced with HT. Individual wells are then screened by ELISA for human anti-PSMA monoclonal IgM and IgG antibodies. Once extensive hybridoma growth occurs, medium is observed usually after 10-14 days. The antibody secreting hybridomas are replated, screened again, and if still positive for human IgG, anti-PSMA monoclonal antibodies, can be subcloned at least twice by limiting dilution. The stable subclones are then cultured *in vitro* to generate small amounts of antibody in tissue culture medium for characterization.

### Characterization of Binding of Human Monoclonal Antibodies to PSMA

To characterize binding of human monoclonal PSMA antibodies of the invention, sera from immunized mice can be tested, for example, by ELISA. Briefly, microtiter plates are coated with purified PSMA at 0.25 µg/ml in PBS, and then blocked with 5% bovine serum albumin in PBS. Dilutions of plasma from PSMA-immunized mice are added to each well and incubated for 1-2 hours at 37°C. The plates are washed with PBS/Tween and then incubated with a goat-anti-human IgG Fc-specific polyclonal reagent conjugated to alkaline phosphatase for 1 hour at 37°C. After washing, the plates are developed with pNPP substrate (1 mg/ml), and analyzed at OD of 405-650. Preferably, mice which develop the highest titers will be used for fusions.

An ELISA assay as described above can also be used to screen for hybridomas that show positive reactivity with PSMA immunogen. Hybridomas that bind with high avidity to PSMA will be subcloned and further characterized. One clone from each hybridoma, which retains the reactivity of the parent cells (by ELISA), can be chosen for making a 5-10 vial cell bank stored at -140°C, and for antibody purification.

To purify human anti-PSMA antibodies, selected hybridomas can be grown in two-liter spinner-flasks for monoclonal antibody purification. Supernatants can be filtered and concentrated before affinity chromatography with protein A-sepharose (Pharmacia, Piscataway, NJ). Eluted IgG can be checked by gel electrophoresis and high performance liquid chromatography to ensure purity. The buffer solution can be exchanged into PBS, and the concentration can be determined by OD₂₈₀ using 1.43 extinction coefficient. The monoclonal antibodies can be aliquoted and stored at -80 °C.

To determine if the selected human anti-PSMA monoclonal antibodies bind to unique epitopes, each antibody can be biotinylated using commercially available reagents (Pierce, Rockford, IL). Competition studies using unlabeled monoclonal antibodies and biotinylated monoclonal antibodies can be performed using PSMA coated-ELISA plates as described above. Biotinylated mAb binding can be detected with a strep-avidin-alkaline phosphatase probe.

To determine the isotype of purified antibodies, isotype ELISAs can be performed. Wells of microtiter plates can be coated with 10 µg/ml of anti-human Ig overnight at 4°C. After blocking with 5% BSA, the plates are reacted with 10 µg/ml of monoclonal antibodies or purified isotype controls, at ambient temperature for two hours. The wells can then be reacted with either human IgGl or human IgM-specific alkaline phosphatase-conjugated probes. Plates are developed and analyzed as described above.

In order to demonstrate binding of monoclonal antibodies to live cells expressing the PSMA, flow cytometry can be used. Briefly, cell lines expressing PSMA (grown under standard growth conditions) are mixed with various concentrations of monoclonal antibodies in PBS containing 0.1% Tween 80 and 20% mouse serum, and incubated at 37°C for 1 hour. After washing, the cells are reacted with Fluorescein-labeled anti-human IgG antibody under the same conditions as the primary antibody staining. The samples can be analyzed by FACScan instrument using light and side scatter properties to gate on single cells. An alternative assay using fluorescence microscopy may be used (in addition to or instead of) the flow cytometry assay. Cells can be stained exactly as described above and examined by fluorescence microscopy. This method allows visualization of individual cells, but may have diminished sensitivity depending on the density of the antigen.

Anti-PSMA human IgGs can be further tested for reactivity with PSMAantigen by Western blotting. Briefly, cell extracts from cells expressing PSMA can be prepared 10 and subjected to sodium dodecyl sulfate (SDS) polyacrylamide gel electrophoresis. After electrophoresis, the separated antigens will be transferred to nitrocellulose membranes, blocked with 20% mouse serum, and probed with the monoclonal antibodies to be tested. Human IgG binding can be detected using anti-human IgG alkaline phosphatase and developed with BCIP/NBT substrate tablets (Sigma Chem. Co., St. Louis, MO).

### Phagocytic and Cell Killing Activities of Human Monoclonal Antibodies to PSMA

In addition to binding specifically to PSMA, human monoclonal anti-PSMA antibodies can be tested for their ability to mediate phagocytosis and killing of cells expressing PSMA. The testing of monoclonal antibody activity *in vitro* will provide an initial screening prior to testing *in vivo* models. Briefly, polymorphonuclear cells (PMN), or other effector cells, from healthy donors can be purified by Ficoll Hypaque density centrifugation, followed by lysis of contaminating erythrocytes. Washed PMNs, can be suspended in RPMI supplemented with 10% heat-inactivated fetal calf serum and mixed with ⁵¹Cr labeled cells expressing PSMA, at various ratios of effector cells to tumor cells (-effector cells:tumor cells). Purified human anti-PSMA IgGs can then be added at various concentrations. Irrelevant human IgG can be used as negative control. Assays can be carried out for 0-120 minutes at 37°C. Samples can be assayed for cytolysis by measuring ⁵¹Cr release into the culture supernatant. Anti-PSMA monoclonal can also be tested in combinations with each other to determine whether cytolysis is enhanced with multiple monoclonal antibodies.

Human monoclonal antibodies which bind to PSMA also can be tested in an *in vivo* model (e.g., in mice) to determine their efficacy in mediating phagocytosis and killing of cells expressing PSMA, e.g., tumor cells. These antibodies can be selected, for example, based on the following criteria, which are not intended to be exclusive:
1.) binding to live cells expressing PSMA;
2.) high affinity of binding to PSMA;
3.) binding to a unique epitope on PSMA (to eliminate the possibility that monoclonal antibodies with complimentary activities when used in combination would compete for binding to the same epitope);
4.) opsonization of cells expressing PSMA;
5.) mediation of growth inhibition, phagocytosis and/or killing of cells expressing PSMA in the presence of human effector cells.

Preferred human monoclonal antibodies of the invention meet one or more, and preferably all, of these criteria. In a particular embodiment, the human monoclonal antibodies are used in combination, e.g., as a pharmaceutical composition comprising two or more anti-PSMA monoclonal antibodies or fragments thereof. For example, human anti-PSMA monoclonal antibodies having different, but complementary activities can be combined in a single therapy to achieve a desired therapeutic or diagnostic effect. An illustration of this would be a composition containing an anti-PSMA human monoclonal antibody that mediates highly effective killing of target cells in the presence of effector cells, combined with another human anti-PSMA monoclonal antibody that inhibits the growth of cells expressing PSMA.

### II. Production of Transgenic Nonhuman Animals Which Generate Human Monoclonal Anti-PSMA Antibodies

In yet another aspect, the invention provides transgenic non-human animals, e.g., a transgenic mice, which are capable of expressing human monoclonal antibodies that specifically bind to PSMA, preferably with high affinity. In a preferred embodiment, the transgenic non-human animals, e.g., the transgenic mice (HuMab mice), have a genome comprising a human heavy chain transgene and a light chain transgene. In one embodiment, the transgenic non-human animals, e.g., the transgenic mice, have been immunized with a purified or enriched preparation of PSMA antigen and/or cells expressing PSMA. Preferably, the transgenic non-human animals, e.g., the transgenic mice, are capable of producing multiple isotypes of human monoclonal antibodies to PSMA (e.g., IgG, IgA and/or IgE) by undergoing V-D-J recombination and isotype switching. Isotype switching may occur by, e.g., classical or non-classical isotype switching.

The design of a transgenic non-human animal that responds to foreign antigen stimulation with a heterologous antibody repertoire, requires that the heterologous immunoglobulin transgenes contain within the transgenic animal function correctly throughout the pathway of B-cell development. In a preferred embodiment, correct function of a heterologous heavy chain transgene includes isotype switching. Accordingly, the transgenes of the invention are constructed so as to produce isotype switching and one or more of the following: (1) high level and cell-type specific expression, (2) functional gene rearrangement, (3) activation of and response to allelic exclusion, (4) expression of a sufficient primary repertoire, (5) signal transduction, (6) somatic hypermutation, and (7) domination of the transgene antibody locus during the immune response.

Not all of the foregoing criteria need be met For example, in those embodiments wherein the endogenous immunoglobulin loci of the transgenic animal are functionally disrupted, the transgene need not activate allelic exclusion. Further, in those embodiments wherein the transgene comprises a functionally rearranged heavy and/or light chain immunoglobulin gene, the second criteria of functional gene rearrangement is unnecessary, at least for that transgene which is already rearranged. For background on molecular immunology, see, Fundamental Immunology, 2nd edition (1989), Paul William E., ed. Raven Press, N.Y., which is incorporated herein by reference.

In certain embodiments, the transgenic non-human animals used to generate the human monoclonal antibodies of the invention contain rearranged, unrearranged or a combination of rearranged and unrearranged heterologous immunoglobulin heavy and light chain transgenes in the germline of the transgenic animal. Each of the heavy chain transgenes comprises at least one C_{H} gene. In addition, the heavy chain transgene may contain functional isotype switch sequences, which are capable of supporting isotype switching of a heterologous transgene encoding multiple C_{H} genes in the B-cells of the transgenic animal. Such switch sequences may be those which occur naturally in the germline immunoglobulin locus from the species that serves as the source of the transgene C_{H} genes, or such switch sequenoes may be derived from those which occur in the species that is to receive the transgene construct (the transgenic animal). For example, a human transgene construct that is used to produce a transgenic mouse may produce a higher frequency of isotype switching events if it incorporates switch sequences similar to those that occur naturally in the mouse heavy chain locus, as presumably the mouse switch sequences are optimized to function with the mouse switch recombinase enzyme system, whereas the human switch sequences are not. Switch sequences may be isolated and cloned by conventional cloning methods, or may be synthesized *de novo* from overlapping synthetic oligonucleotides designed on the basis of published sequence information relating to immunoglobulin switch region sequences (Mills et al., Nucl. Acids Res. 15:7305-7316 (1991); Sideras et al., Inrl. Immunol. 1:631-642 (1989), which are incorporated herein by reference). For each of the foregoing transgenic animals, functionally rearranged heterologous heavy and light chain immunoglobulin transgenes are found in a significant fraction of the B-cells of the transgenic animal (at least 10 percent).

The transgenes used to generate the transgenic animals of the invention include a heavy chain transgene comprising DNA encoding at least one variable gene segment, one diversity gene segment, one joining gene segment and at least one constant region gene segment. The immunoglobulin light chain transgene comprises DNA encoding at least one variable gene segment, one joining gene segment and at least one constant region gene segment. The gene segments encoding the light and heavy chain gene segments are heterologous to the transgenic non-human animal in that they are derived from, or correspond to, DNA encoding immunoglobulin heavy and light chain gene segments from a species not consisting of the transgenic non-human animal. In one aspect of the invention, the transgene is constructed such that the individual gene segments are unrearranged, i.e., not rearranged so as to encode a functional immunoglobulin light or heavy chain. Such unrearranged transgènes support recombination of the V, D, and J gene segments (functional rearrangement) and preferably support incorporation of all or a portion of a D region gene segment in the resultant rearranged immunoglobulin heavy chain within the transgenic non-human animal when exposed to PSMA antigen.

In an alternate embodiment, the transgenes comprise an unrearranged "mini-locus". Such transgenes typically comprise a substantial portion of the C, D, and J segments as well as a subset of the V gene segments. In such transgene constructs, the various regulatory sequences, e.g. promoters, enhancers, class switch regions, splice-donor and splice-acceptor sequences for RNA processing, recombination signals and the like, comprise corresponding sequences derived from the heterologous DNA. Such regulatory sequences may be incorporated into the transgene from the same or a related species of the non-human animal used in the invention. For example, human immunoglobulin gene segments may be combined in a transgene with a rodent immunoglobulin enhancer sequence for use in a transgenic mouse. Alternatively, synthetic regulatory sequences may be incorporated into the transgene, wherein such synthetic regulatory sequences are not homologous to a functional DNA sequence that is known to occur naturally in the genomes of mammals. Synthetic regulatory sequences are designed according to consensus rules, such as, for example, those specifying the permissible sequences of a splice-acceptor site or a promoter/enhancer motif. For example, a minilocus comprises a portion of the genomic immunoglobulin locus having at least one internal (i.e., not at a terminus of the portion) deletion of a non-essential DNA portion (e.g., intervening sequence; intron or portion thereof) as compared to the naturally-occurring germline Ig locus.

In a preferred embodiment of the invention, the transgenic animal used to generate human antibodies to PSMA contains at least one, typically 2-10, and sometimes 25-50 or more copies of the transgene described in Example 12 of WO 98/24884 (e.g., pHC1 or pHC2) bred with an animal containing a single copy of a light chain transgene described in Examples 5, 6, 8, or 14 of WO 98/24884, and the offspring bred with the J_{H} deleted animal described in Example 10 of WO 98/24884, the contents of which are hereby expressly incorporated by reference. Animals are bred to homozygosity for each of these three traits. Such animals have the following genotype: a single copy (per haploid set of chromosomes) of a human heavy chain unrearranged mini-locus (described in Example 12 of WO 98/24884), a single copy (per haploid set of chromosomes) of a rearranged human K light chain construct (described in Example 14 of WO 98/24884), and a deletion at each endogenous mouse heavy chain locus that removes all of the functional J_{H} segments (described in Example 10 of WO 98/24884). Such animals are bred with mice that are homozygous for the deletion of the J_{H} segments (Examples 10 of WO 98/24884) to produce offspring that are homozygous for the J_{H} deletion and hemizygous for the human heavy and light chain constructs. The resultant animals are injected with antigens and used for production of human monoclonal antibodies against these antigens.

B cells isolated from such an animal are monospecific with regard to the human heavy and light chains because they contain only a single copy of each gene. Furthermore, they will be monospecific with regards to human or mouse heavy chains because both endogenous mouse heavy chain gene copies are nonfunetional by virtue of the deletion spanning the J_{H} region introduced as described in Example 9 and 12 of WO 98/24884. Furthermore, a substantial fraction of the B cells will be monospecific with regards to the human or mouse light chains because expression of the single copy of the rearranged human κ light chain gene will allelically and isotypically exclude the rearrangement of the endogenous mouse κ and lambda chain genes in a significant fraction of B-cells.

The transgenic mouse of the preferred embodiment will exhibit immunoglobulin production with a significant repertoire, ideally substantially similar to that of a native mouse. Thus, for example, in embodiments where the endogenous Ig genes have been inactivated, the total immunoglobulin levels will range from about 0.1 to 10 mg/ml of serum, preferably 0. 5 to 5 mg/ml, ideally at least about 1.0 mg/ml. When a transgene capable of effecting a switch to IgG from IgM has been introduced into the transgenic mouse, the adult mouse ratio of serum IgG to IgM is preferably about 10:1. The IgG to IgM ratio will be much lower in the immature mouse. In general, greater than about 10%, preferably 40 to 80% of the spleen and lymph node B cells express exclusively human IgG protein.

The repertoire will ideally approximate that shown in a non-transgenic mouse, usually at least about 10% as high, preferably 25 to 50% or more. Generally, at least about a thousand different immunoglobulins (ideally IgG), preferably 10⁴ to 10⁶ or more, will be produced, depending primarily on the number of different V, J and D regions introduced into the mouse genome. These immunoglobulins will typically recognize about one-half or more of highly antigenic proteins, e.g., staphylococcus protein A. Typically, the immunoglobulins will exhibit an affinity for preselected antigens of at least about 10⁷M⁻¹, preferably at least about 10⁹M⁻¹, more preferably at least about 10¹⁰M⁻¹,10¹¹M⁻¹, 10¹²M⁻¹, or greater, e.g., up to 10¹³M⁻¹ or greater.

In some embodiments, it may be preferable to generate mice with predetermined repertoires to limit the selection of V genes represented in the antibody response to a predetermined antigen type. A heavy chain transgene having a predetermined repertoire may comprise, for example, human VH genes which are preferentially used in antibody responses to the predetermined antigen type in humans. Alternatively, some VH genes may be excluded from a defined repertoire for various reasons (e.g., have a low likelihood of encoding high affinity V regions for the predetermined antigen; have a low propensity to undergo somatic mutation and affinity sharpening; or are immunogenic to certain humans). Thus, prior to rearrangement of a transgene containing various heavy or light chain gene segments, such gene segments may be readily identified, e.g. by hybridization or DNA sequencing, as being from a species of organism other than the transgenic animal.

The transgenic mice of the present invention can be immunized with a purified or enriched preparation of PSMA antigen and/or cells expressing PSMA as described previously. The mice will produce B cells which undergo class-switching via intratransgene switch recombination (cis-switching) and express immunoglobulins reactive with PSMA. The immunoglobulins can be human sequence antibodies, wherein the heavy and light chain polypeptides are encoded by human transgene sequences, which may include sequences derived by somatic mutation and V region recombinatorial joints, as well as germline-encoded sequences; these human sequence immunoglobulins can be referred to as being substantially identical to a polypeptide sequence encoded by a human V_{L} or V_{H} gene segment and a human J_{L} or J_{L} segment, even though other non-germline sequences may be present as a result of somatic mutation and differential V-J and V-D-J recombination joints. With respect to such human sequence antibodies, the variable regions of each chain are typically at least 80 percent encoded by human germline V, J, and, in the case of heavy chains, D, gene segments; frequently at least 85 percent of the variable regions are encoded by human germline sequences present on the transgene; often 90 or 95 percent or more of the variable region sequences are encoded by human germline sequences present on the transgene. However, since non-germline sequences are introduced by somatic mutation and VJ and VDJ joining, the human sequence antibodies will frequently have some variable region sequences (and less frequently constant region sequences) which are not encoded by human V, D, or J gene segments as found in the human transgene(s) in the germline of the mice. Typically, such non-germline sequences (or individual nucleotide positions) will cluster in or near CDRs, or in regions where somatic mutations are known to cluster.

The human sequence antibodies which bind to the predetermined antigen can result from isotype switching, such that human antibodies comprising a human sequence γ chain (such as γl, γ2a, γ2B, or γ3) and a human sequence light chain (such as K) are produced. Such isotype-switched human sequence antibodies often contain one or more somatic mutation(s), typically in the variable region and often in or within about 10 residues of a CDR) as a result of affinity maturation and selection of B cells by antigen, particularly subsequent to secondary (or subsequent) antigen challenge. These high affinity human sequence antibodies may have binding affinities of at least 1 x 10⁹ M⁻¹, typically at least 5 x 10⁹ M⁻¹, frequently more than 1 x 10¹⁰ M⁻¹, and sometimes 5 x 10¹⁰ M⁻¹ to 1 x 10¹¹ M⁻¹ or greater.

Another aspect of the invention pertains to the B cells from such mice which can be used to generate hybridomas expressing human monoclonal antibodies which bind with high affinity (e.g., greater than 2 x 10⁹ M⁻¹) to PSMA. Thus, in another embodiment of the invention, these hybridomas are used to generate a composition comprising an immunoglobulin having an affinity constant (Ka) of at least 2 x 10⁹ M⁻¹ for binding PSMA, wherein said immunoglobulin comprises:
a human sequence light chain composed of (1) a light chain variable region having a polypeptide sequence which is substantially identical to a polypeptide sequence encoded by a human V_{L} gene segment and a human J_{L} segment, and (2) a light chain constant region having a polypeptide sequence which is substantially identical to a polypeptide sequence encoded by a human C_{L} gene segment; and
a human sequence heavy chain composed of a (1) a heavy chain variable region having a polypeptide sequence which is substantially identical to a polypeptide sequence encoded by a human V_{H} gene segment, optionally a D region, and a human J_{H} segment, and (2) a constant region having a polypeptide sequence which is substantially identical to a polypeptide sequence encoded by a human C_{H} gene segment.

The development of high affinity human monoclonal antibodies against PSMA is facilitated by a method for expanding the repertoire of human variable region gene segments in a transgenic mouse having a genome comprising an integrated human immunoglobulin transgene, said method comprising introducing into the genome a V gene transgene comprising V region gene segments which are not present in said integrated human immunoglobulin transgene. Often, the V region transgene is a yeast artificial chromosome comprising a portion of a human V_{H} or V_{L} (V_{K}) gene segment array, as may naturally occur in a human genome or as may be spliced together separately by recombinant methods, which may include out-of-order or omitted V gene segments. Often at least five or more functional V gene segments are contained on the YAC. In this variation, it is possible to make a transgenic mouse produced by the V repertoire expansion method, wherein the mouse expresses an immunoglobulin chain comprising a variable region sequence encoded by a V region gene segment present on the V region transgene and a C region encoded on the human Ig transgene. By means of the V repertoire expansion method, transgenic mice having at least 5 distinct V genes can be generated; as can mice containing at least about 24 V genes or more. Some V gene segments may be non-functional (e.g., pseudogenes and the like); these segments may be retained or may be selectively deleted by recombinant methods available to the skilled artisan, if desired.

Once the mouse germline has been engineered to contain a functional YAC having an expanded V segment repertoire, substantially not present in the human Ig transgene containing the J and C gene segments, the trait can be propagated and bred into other genetic backgrounds, including backgrounds where the functional YAC having an expanded V segment repertoire is bred into a mouse germline having a different human Ig transgene. Multiple functional YACs having an expanded V segment repertoire may be bred into a germline to work with a human Ig transgene (or multiple human Ig transgenes). Although referred to herein as YAC transgenes, such transgenes when integrated into the genome may substantially lack yeast sequences, such as sequences required for autonomous replication in yeast; such sequences may optionally be removed by genetic engineering (e.g., restriction digestion and pulsed-field gel electrophoresis or other suitable method) after replication in yeast in no longer necessary (i.e., prior to introduction into a mouse ES cell or mouse prorygote). Methods of propagating the trait of human sequence immunoglobulin expression, include breeding a transgenic mouse having the human Ig transgene(s), and optionally also having a functional YAC having an expanded V segment repertoire. Both V_{H} and V_{L} gene segments may be present on the YAC. The transgenic mouse may be bred into any background desired by the practitioner, including backgrounds harboring other human transgenes, including human Ig transgenes and/or transgenes encoding other human lymphocyte proteins. The invention also provides a high affinity human sequence immunoglobulin produced by a transgenic mouse having an expanded V region repertoire YAC transgene. Although the foregoing describes a preferred embodiment of the transgenic animal of the invention, other embodiments are contemplated which have been classified in four categories:
I. Transgenic animals containing an unrearranged heavy and rearranged light immunoglobulin transgene;
II. Transgenic animals containing an unrearranged heavy and unrearranged light immunoglobulin transgene;
III. Transgenic animal containing rearranged heavy and an unrearranged light immunoglobulin transgene; and
IV. Transgenic animals containing rearranged heavy and rearranged light immunoglobulin transgenes.

Of these categories of transgenic animal, the preferred order of preference is as follows II > I > III > IV where the endogenous light chain genes (or at least the K gene) have been knocked out by homologous recombination (or other method) and I > II > III >IV where the endogenous light chain genes have not been knocked out and must be dominated by allelic exclusion.

### III. Bispecific/ Multispecific Molecules Which Bind to PSMA

In yet another embodiment of the invention, human monoclonal antibodies to PSMA, or antigen-binding portions thereof, can be derivatized or linked to another functional molecule, e.g., another peptide or protein (e.g., an Fab' fragment) to generate a bispecific or multispecific molecule which binds to multiple binding sites or target epitopes. For example, an antibody or antigen-binding portion of the invention can be functionally linked (e.g., by chemical coupling, genetic fusion, noncovalent association or otherwise) to one or more other binding molecules, such as another antibody, antibody fragment, peptide or binding mimetic.

Accordingly, the present invention includes bispecific and multispecific molecules comprising at least one first binding specificity for PSMA and a second binding specificity for a second target epitope. In a particular embodiment of the invention, the second target epitope is an Fc receptor, e.g., human FcγRI (CD64) or a human Fcα receptor (CD89). Therefore, the invention includes bispecific and multispecific molecules capable of binding both to FcγR, FcαR or FcεR expressing effector cells (e.g., monocytes, macrophages or polymorphonuclear cells (PMNs)), and to target cells expressing PSMA. These bispecific and multispecific molecules target PSMA expressing cells to effector cell and, like the human monoclonal antibodies of the invention, trigger Fc receptor-mediated effector cell activities, such as phagocytosis of a PSMA expressing cells, antibody dependent cell-mediated cytotoxicity (ADCC), cytokine release, or generation of superoxide anion.

Bispecific and multispecific molecules of the invention can further include a third binding specificity, in addition to an anti-Fc binding specificity and an anti-PSMA binding specificity. In one embodiment, the third binding specificity is an anti-enhancement factor (EF) portion, e.g., a molecule which binds to a surface protein involved in cytotoxic activity and thereby increases the immune response against the target cell. The "anti-enhancement factor portion" can be an antibody, functional antibody fragment or a ligand that binds to a given molecule, e.g., an antigen or a receptor, and thereby results in an enhancement of the effect of the binding determinants for the F_{c} receptor or target cell antigen. The "anti-enhancement factor portion" can bind an F_{c} receptor or a target cell antigen. Alternatively, the anti-enhancement factor portion can bind to an entity that is different from the entity to which the first and second binding specificities bind. For example, the anti-enhancement factor portion can bind a cytotoxic T-cell (e.g. via CD2, CD3, CD8, CD28, CD4, CD40, ICAM-I or other immune cell that results in an increased immune response against the target cell).

In one embodiment, the bispecific and multispecific molecules of the invention comprise as a binding specificity at least one antibody, or an antibody fragment thereof, including, e.g., an Fab, Fab', F(ab')₂, Fv, or a single chain Fv. The antibody may also be a light chain or heavy chain dimer, or any minimal fragment thereof such as a Fv or a single chain construct as described in Ladner et al. U.S. Patent No. 4,946,778, issued August 7,1990, the contents of which is expressly incorporated by reference.

In one embodiment bispecific and multispecific molecules of the invention comprise a binding specificity for an FcγR or an FcαR present on the surface of an effector cell, and a second binding specificity for a target cell antigen, e.g., PSMA.

In one embodiment, the binding specificity for an Fc receptor is provided by a human monoclonal antibody, the binding of which is not blocked by human immunoglobulin G (IgG. As used herein, the term "IgG receptor" refers to any of the eight γ-chain genes located on chromosome 1. These genes encode a total of twelve transmembrane or soluble receptor isoforms which are grouped into three Fcγ receptor classes: FcγRI (CD64), FcγRII(CD32), and FcγRIII (CD16). In one preferred embodiment, the Fcγ receptor a human high affinity FcγRI. The human FcγRI is a 72 kDa molecule, which shows high affinity for monomeric IgG (10⁸ - 10⁹M⁻¹).

The production and characterization of these preferred monoclonal antibodies are described by Fanger et al, in PCT application WO 88/00052 and in U.S. Patent No. 4,954,617, the teachings of which are fully incorporated by reference herein. These antibodies bind to an epitope of FcγRI, FcγRII or FcγRIII at a site which is distinct from the Fcγ binding site of the receptor and, thus, their binding is not blocked substantially by physiological levels of IgG. Specific anti-FcγRI antibodies useful in this invention are mAb 22, mAb 32, mAb 44, mAb 62 and mAb 197. The hybridoma producing mAb 32 is available from the American Type Culture Collection, ATCC Accession No. HB9469. Anti-FcγRI mAb 22, F(ab')₂ fragments of mAb 22, and can be obtained from Medarex, Inc. (Annandale, N.J.). In other embodiments, the anti-Fcγ receptor antibody is a humanized form of monoclonal antibody 22 (H22). The production and characterization of the H22 antibody is described in Graziano, R.F. et al. (1995) J. Immunol 155 (10): 4996-5002and PCT/US93/10384. The H22 antibody producing cell line was deposited at the American Type Culture Collection on November 4, 1992 under the designation HA022CL1 and has the accession no. CRL 11177.

In still other preferred embodiments, the binding specificity for an Fc receptor is provided by an antibody that binds to a human IgA receptor, e.g., an Fc-alpha receptor (FcαRI (CD89)), the binding of which is preferably not blocked by human immunoglobulin A (IgA). The term "IgA receptor" is intended to include the gene product of one α-gene (FcαRI) located on chromosome 19. This gene is known to encode several alternatively spliced transmembrane isoforms of 55 to 110 kDa. FcαRI (CD89) is constitutively expressed on monocytes/macrophages, eosinophilic and neutrophilic granulocytes, but not on non-effector cell populations. FcαRI has medium affinity (≈ 5 × 10⁷ M⁻¹) for both IgA1 and IgA2, which is increased upon exposure to cytokines such as G-CSF or GM-CSF (Morton, H.C. et al. (1996) Critical Reviews in Immunology 16:423-440). Four FcαRI-specific monoclonal antibodies, identified as A3, A59, A62 and A77, which bind FcαRI outside the IgA ligand binding domain, have been described (Monteiro, R.C. et al., 1992, J. Immunol. 148:1764).

FcαRI and FcγRI are preferred trigger receptors for use in the invention because they are (1) expressed primarily on immune effector cells, e.g., monocytes, PMNs, macrophages and dendritic cells; (2) expressed at high levels (e.g., 5,000-100,000 per cell); (3) mediators of cytotoxic activities (e.g., ADCC, phagocytosis); (4) mediate enhanced antigen presentation of antigens, including self-antigens, targeted to them.

In other embodiments, bispecific and multispecific molecules of the invention further comprise a binding specificity which recognizes, e.g., binds to, a target cell antigen, e.g., PSMA. In a preferred embodiment, the binding specificity is provided by a human monoclonal antibody of the present invention.

An "effector cell specific antibody" as used herein refers to an antibody or functional antibody fragment that binds the Fc receptor of effector cells. Preferred antibodies for use in the subject invention bind the Fc receptor of effector cells at a site which is not bound by endogenous immunoglobulin.

As used herein, the term "effector cell" refers to an immune cell which is involved in the effector phase of an immune response, as opposed to the cognitive and activation phases of an immune response. Exemplary immune cells include a cell of a myeloid or lymphoid origin, e.g., lymphocytes (e.g., B cells and T cells including cytolytic T cells (CTLs)), killer cells, natural killer cells, macrophages, monocytes, eosinophils, neutrophils, polymorphonuclear cells, granulocytes, mast cells, and basophils. Some effector cells express specific Fc receptors and carry out specific immune functions. In preferred embodiments, an effector cell is capable of inducing antibody-dependent cell-mediated cytotoxicity (ADCC), e.g., a neutrophil capable of inducing ADCC. For example, monocytes, macrophages, which express FcR are involved in specific killing of target cells and presenting antigens to other components of the immune system, or binding to cells that present antigens. In other embodiments, an effector cell can phagocytose a target antigen, target cell, or microorganism. The expression of a particular FcR on an effector cell can be regulated by humoral factors such as cytokines. For example, expression of FcγRI has been found to be up-regulated by interferon gamma (IFN-γ). This enhanced expression increases the cytotoxic activity of FcγRI-bearing cells against targets. An effector cell can phagocytose or lyse a target antigen or a target cell.

"Target cell" shall mean any undesirable cell in a subject (e.g., a human or animal) that can be targeted by a composition (e.g., a human monoclonal antibody, a bispecific or a multispecific molecule) of the invention. In preferred embodiments, the target cell is a cell expressing or overexpressing PSMA. Cells expressing PSMA typically include tumor cells, such as prostate, renal and colon tumor cells.

While human monoclonal antibodies are preferred, other antibodies which can be employed in the bispecific or multispecifc molecules of the invention are murine, chizneric and humanized monoclonal antibodies.

Chimeric mouse-human monoclonal antibodies (i.e., chimeric antibodies) can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted. (see Robinson et al., International Patent Publication PCT/US86/02269; Akiza, et al., European Patent Application 184,187; Taniguchi, M., European Patent Application 171,496; Morrison et al., European Patent Application 173,494; Neuberger et al., International Application WO 86/01533; Cabilly et al. U.S. Patent No. 4,816,567; Cabilly et al., European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Canc. Res. 47:999-1005; Wood et al. (1985) Nature 314:446-449; and Shaw et al., 1988, J. Natl Cancer Inst. 80:1553-1559).

The chimeric antibody can be further humanized by replacing sequences of the Fv variable region which are not directly involved in antigen binding with equivalent sequences from human Fv variable regions. General reviews of humanized chimeric antibodies are provided by Morrison, S. L.,1985, Science 229:1202-1207 and by Oi et al., 1986, BioTechniques 4:214. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from 7E3, an anti-GPII_{b}IIIₐ antibody producing hybridoma. The recombinant DNA encoding the chimeric antibody, or fragment thereof, can then be cloned into an appropriate expression vector. Suitable humanized antibodies can alternatively be produced by CDR substitution U.S. Patent 5,225,539; Jones et al. 1986 Nature 321:552-525; Verhoeyan et al. 1988 Science 239:1534; and Beidler et al. 1988 J. Immunol. 141:4053-4060.

All of the CDRs of a particular human antibody may be replaced with at least a portion of a non-human CDR or only some of the CDRs may be replaced with non-human CDRs. It is only necessary to replace the number of CDRs required for binding of the humanized antibody to the Fc receptor.

An antibody can be humanized by any method, which is capable of replacing at least a portion of a CDR of a human antibody with a CDR derived from a non-human antibody. Winter describes a method which may be used to prepare the humanized antibodies of the present invention (UK Patent Application GB 2188638A, filed on March 26, 1987), the contents of which is expressly incorporated by reference. The human CDRs may be replaced with non-human CDRs using oligonucleotide site-directed mutagenesis as described in International Application WO 94/10332 entitled, *Humanized Antibodies to Fc Receptors for Immunoglobulin G on Human Mononuclear Phagocytes.*

Also within the scope of the invention are chimeric and humanized antibodies in which specific amino acids have been substituted, deleted or added. In particular, preferred humanized antibodies have amino acid substitutions in the framework region, such as to improve binding to the antigen. For example, in a humanized antibody having mouse CDRs, amino acids located in the human framework region can be replaced with the amino acids located at the corresponding positions in the mouse antibody. Such substitutions are known to improve binding of humanized antibodies to the antigen in some instances. Antibodies in which amino acids have been added, deleted, or substituted are referred to herein as modified antibodies or altered antibodies.

The term modified antibody is also intended to include antibodies, such as monoclonal antibodies, chimeric antibodies, and humanized antibodies which have been modified by, e.g., deleting, adding, or substituting portions of the antibody. For example, an antibody can be modified by deleting the constant region and replacing it with a constant region meant to increase half-life, e.g., serum half-life, stability or affinity of the antibody. Any modification is within the scope of the invention so long as the bispecific and multispecific molecule has at least one antigen binding region specific for an FcγR and triggers at least one effector function.

Bispecific and multispecific molecules of the present invention can be made using chemical techniques (see e.g., D. M. Kranz et al. (1981) Proc. Natl. Acad. Sci. USA 78:5807), "polydoma" techniques (See U.S. Patent 4,474,893, to Reading), or recombinant DNA techniques.

In particular, bispecific and multispecific molecules of the present invention can be prepared by conjugating the constituent binding specificities, e.g., the anti-FcR and anti-PSMA binding specificities, using methods known in the art and described in the examples provided herein. For example, each binding specificity of the bispecific and multispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-S-acetyl-thioacetate (SATA), 5,5'-dithiobis(2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohaxane-1-carboxylate (sulfo-SMCC) (see e.g., Karpovsky et al. (1984) J. Exp. Med. 160:1686; Liu, MA et al. (1985) Proc. Natl. Acad. Sci. USA 82:8648). Other methods include those described by Paulus (Behring Ins. Mitt. (1985) No. 78,118-132); Brennan et al. (Science (1985) 229:81-83), and Glennie et al. (J. Immunol. (1987) 139: 2367-2375). Preferred conjugating agents are SATA and sulfo-SMCC, both available from Pierce Chemical Co. (Rockford, IL).

When the binding specificities are antibodies (e.g., two humanized antibodies), they can be conjugated via sulfhydryl bonding of the C-terminus hinge regions of the two heavy chains. In a particularly preferred embodiment, the hinge region is modified to contain an odd number of sulfhydryl residues, preferably one, prior to conjugation.

Alternatively, both binding specificities can be encoded in the same vector and expressed and assembled in the same host cell. This method is particularly useful where the bispecific and multispecific molecule is a mAb x mAb, mAb x Fab, Fab x F(ab')₂ or ligand x Fab fusion protein. A bispecific and multispecific molecule of the invention, e.g., a bispecific molecule can be a single chain molecule, such as a single chain bispecific antibody, a single chain bispecific molecule comprising one single chain antibody and a binding determinant, or a single chain bispecific molecule comprising two binding determinants. Bispecific and multispecific molecules can also be single chain molecules or may comprise at least two single chain molecules. Methods for preparing bi- and multspecific molecules are described for example in U.S. Patent Number 5,260,203; U.S. Patent Number 5,455,030; U.S. Patent Number 4,881,175; U.S. Patent Number 5,132,405; U.S. Patent Number 5,091,513; U.S. Patent Number 5,476,786; U.S. Patent Number 5,013,653; U.S. PatentNumber 5,258,498; and U.S. Patent Number 5,482,858.

Binding of the bispecific and multispecific molecules to their specific targets can be confirmed by enzyme-linked immunosorbent assay (ELISA), a radioimmunoassay (RIA), FACS analysis, a bioassay (e.g., growth inhibition), or a Western Blot Assay. Each of these assays generally detects the presence of protein-antibody complexes of particular interest by employing a labeled reagent (e.g., an antibody) specific for the complex of interest For example, the FcR-antibody complexes can be detected using e.g., an enzyme-linked antibody or antibody fragment which recognizes and specifically binds to the antibody-FcR complexes. Alternatively, the complexes can be detected using any of a variety of other immunoassays. For example, the antibody can be radioactively labeled and used in a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a γ counter or a scintillation counter or by autoradiography.

### IV. Antibody Conjugates/Immunotoxins

In another aspect, the present invention features a human anti-PSMA monoclonal antibody, or a fragment thereof, conjugated to a therapeutic moiety, such as a cytotoxin, a drug or a radioisotope. When conjugated to a cytotoxin, these antibody conjugates are referred to as "immunotoxins." A cytotoxin or cytotoxic agent includes any agent that is detrimental to (e.g., kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). An antibody of the present invention can be conjugated to a radioisotope, e.g., radioactive iodine, to generate cytotoxic radiopharmaceuticals for treating a PSMA-related disorder, such as a cancer.

The antibody conjugates of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-γ, or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, e.g., Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies'84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

### V. Pharmaceutical Compositions

In another aspect, the present invention provides a composition, e.g., a pharmaceutical composition, containing one or a combination of human monoclonal antibodies, or antigen-binding portion(s) thereof, of the present invention, formulated together with a pharmaceutically acceptable carrier. In a preferred embodiment, the compositions include a combination of multiple (e.g., two or more) isolated human antibodies or antigen-binding portions thereof of the invention. Preferably, each of the antibodies or antigen-binding portions thereof of the composition binds to a distinct, preselected epitope of PSMA.

In one embodiment, human auti-PSMA monoclonal antibodies having complementary activities are used in combination, e.g., as a pharmaceutical composition, comprising two or more human anti-PSMA monoclonal antibodies. For example, a human monoclonal antibody that mediates highly effective killing of target cells in the presence of effector cells can be combined with another human monoclonal antibody that inhibits the growth of cells expressing PSMA.

In another embodiment, the composition comprises one or a combination of bispecific or multispecifie molecules of the invention (e.g., which contains at least one binding specificity for an Fc receptor and at least one binding specificity for PSMA).

Pharmaceutical compositions of the invention also can be administered in combination therapy, i.e., combined with other agents. For example, the combination therapy can include a composition of the present invention with at least one anti-tumor agent or other conventional therapy.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g., by injection or infusion). Depending on the route of administration, the active compound, i.e., antibody, bispecific and multispecific molecule, may be coated in a material to protect the compound from the action of acids and other natural conditions that may inactivate the compound.

A "pharmaceutically acceptable salt" refers to a salt that retains the desired biological activity of the parent compound and does not impart any undesired toxicological effects (see e.g., Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A composition of the present invention can be administered by a variety of methods known in the art. As will be appreciated by the skilled artisan, the route and/or mode of administration will vary depending upon the desired results. The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J.R. Robinson, ed., Marcel Dekker, Inc., New York, 1978.

To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al. (1984) J. Neuroimmunol. 7:27).

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, soxbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

For the therapeutic compositions, formulations of the present invention include those suitable for oral, nasal, topical (including buccal and sublingual), rectal, vaginal and/or parenteral administration. The formulations may conveniently be presented in unit dosage form and may be prepared by any methods known in the art of pharmacy. The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Ceneraily, out of one hundred per cent, this amount will range from about 0.01 per cent to about ninety-nine percent of active ingredient, preferably from about 0.1 per cent to about 70 per cent, most preferably from about 1 per cent to about 30 per cent

Formulations of the present invention which are suitable for vaginal administration also include pessaries, tampons, creams, gels, pastes, foams or spray fornnulations containing such carriers as are known in the art to be appropriate. Dosage forms for the topical or transdermal administration of compositions of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal epidural and intrastemal injection and infusion.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

When the compounds of the present invention are administered as pharmaceuticals, to humans and animals, they can be given alone or as a pharmaceutical composition containing, for example, 0.01 to 99.5% (more preferably, 0.1 to 90%) of active ingredient in combination with a pharmaceutically acceptable carrier.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved. In general, a suitable daily dose of a compositions of the invention will be that amount of the compound which is the lowest dose effective to produce a therapeutic effect Such an effective dose will generally depend upon the factors described above. It is preferred that administration be intravenous, intramuscular, intraperitoneal, or subcutaneous, preferably administered proximal to the site of the target. If desired, the effective daily dose of a therapeutic compositions may be administered as two, three, four, five, six or more sub-doses administered separately at appropriate intervals throughout the day, optionally, in unit dosage forms. While it is possible for a compound of the present invention to be administered alone, it is preferable to administer the compound as a pharmaceutical formulation (composition).

Therapeutic compositions can be administered with medical devices known in the art. For example, in a preferred embodiment, a therapeutic composition of the invention can be administered with a needleless hypodermic injection device, such as the devices disclosed inU.S. PatentNos. 5,399,163; 5,333,851; 5,312,335; 5,064,413; 4,941,880; 4,790,824; or 4,596,556. Examples of well-known implants and modules useful in the present invention include: U.S. Patent No. 4,487,603, which discloses an implantable micro-infusion pump for dispensing medication at a controlled rate; U.S. Patent No. 4,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. These patents are incorporated herein by reference. Many other such implants, delivery systems, and modules are known to those skilled in the art.

In certain embodiments, the human monoclonal antibodies of the invention can be formulated to ensure proper distribution *in vivo.* For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, e.g., U.S. Patents 4,522,811; 5,374,548; and 5,399,331. The liposomes may comprise one or more moieties which are selectively transported into specific cells or organs, thus enhance targeted drug delivery (*see, e.g.,* V.V. Ranade (1989) J. Clin. Pharmacol. 29:685). Exemplary targeting moieties include folate or biotin (see, e.g., U.S. Patent 5,416,016 to Low et al.); mannosides (Umezawa et al., (1988) Biochem Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134), different species of which may comprise the formulations of the inventions, as well as components of the invented molecules; p120 (Schreier et al. (1994) J. Biol. Chem. 269:9090); see also K. Keinanen; M.L. Laukkanen (1994) FEBSLett. 346:123; J.J. Killion; I.J. Fidler (1994) Immunomethods 4:273. In one embodiment of the invention, the therapeutic compounds of the invention are formulated in liposomes; in a more preferred embodiment, the liposomes include a targeting moiety. In a most preferred embodiment, the therapeutic compounds in the liposomes are delivered by bolus injection to a site proximal to the tumor or infection. The composition must be fluid to the extent that easy syringability exists. It must be stable under the conditions of manufacture and storage and must be preserved against the contaminating action of microorganisms such as bacteria and fungi.

A "therapeutically effective dosage" preferably inhibits tumor growth by at least about 20%, more preferably by at least about 40%, even more preferably by at least about 60%, and still more preferably by at least about 80% relative to untreated subjects. The ability of a compound to inhibit cancer can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

The composition must be sterile and fluid to the extent that the composition is deliverable by syringe. In addition to water, the carrier can be an isotonic buffered saline solution, ethanol, polyol (for example, glycerol, propylene glycol, and liquid polyetheylene glycol, and the like), and suitable mixtures thereof. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as manitol or sorbitol, and sodium chloride in the composition. Long-term absorption of the injectable compositions can be brought about by including in the composition an agent which delays absorption, for example, aluminum monostearate or gelatin.

When the active compound is suitably protected, as described above, the compound may be orally administered, for example, with an inert diluent or an assimilable edible carrier.

### VI. Uses and Methods of the Invention

The compositions (e.g., human monoclonal antibodies to PSMA and derivatives/conjugates thereof) of the present invention have *in vitro* and *in* vivo diagnostic and therapeutic utilities. For example, these molecules can be administered to cells in culture, e.g. *in vitro* or *ex vivo*, or in a subject, e.g., *in vivo,* to treat, prevent or diagnose a variety of disorders. As used herein, the term "subject" is intended to include human and non-human animals. Preferred human animals include a human patient having disorder characterized by expression, typically aberrant expression (e.g., overexpression) of PSMA. For example, the methods and compositions of the present invention can be used to treat a subject with a tumorigenic disorder, e.g., a disorder characterized by the presence of tumor cells expressing PSMA including, for example, prostate, colon and renal (kidney) tumor cells. The term "non-human animals" of the invention includes all vertebrates, e.g., mammals and non-mammals, such as non-human primates, sheep, dog, cow, chickens, amphibians, reptiles, etc.

The compositions (e.g., human antibodies, multispecific and bispecific molecules) of the invention can be initially tested for binding activity associated with therapeutic or diagnostic use *in vitro.* For example, compositions of the invention can be tested using the ELISA and flow cytometric assays described in the Examples below. Moreover, the activity of these molecules in triggering at least one effector-mediated effector cell activity, including cytolysis of cells expressing PSMA can be assayed. Protocols for assaying for effector cell-mediated phagocytosis are described in the Examples below.

The compositions (e.g., human antibodies, multispecific and bispecific molecules) of the invention have additional utility in therapy and diagnosis of PSMA-related diseases. For example, the human monoclonal antibodies, the multispecific or bispecific molecules can be used, for example, to elicit in vivo or *in vitro* one or more of the following biological activities: to opsonize a cell expressing PSMA; to mediate phagocytosis or cytolysis of a cell expressing PSMA in the presence of human effector cells; or to inhibit the growth of a cell expressing PSMA.

In a particular embodiment, the human antibodies and derivatives thereof are used *in vivo* to treat, prevent or diagnose a variety of PSMA-related diseases. Examples of PSMA-related diseases include a variety of cancers, such as prostate, kidney and colon cancer.

Methods of administering the compositions (e.g., human antibodies, multispecific and bispecific molecules) of the invention are known in the art. Suitable dosages of the molecules used will depend on the age and weight of the subject and the particular drug used. The molecules can be coupled to radionuclides, such as 131I, 90Y, 105Rh, etc., as described in Goldenberg, D.M. et al. (1981) Cancer Res. 41: 4354-4360, and in EP 0365 997. The compositions (e.g., human antibodies, multispecific and bispecific molecules) of the invention can also be coupled to anti-infectious agents.

Target-specific effector cells, e.g., effector cells linked to compositions (e.g., human antibodies, multispecific and bispecific molecules) of the invention can also be used as therapeutic agents. Effector cells for targeting can be human leukocytes such as macrophages, neutrophils or monocytes. Other cells include eosinophils, natural killer cells and other IgG- or IgA-receptor bearing cells. If desired, effector cells can be obtained from the subject to be treated. The target-specific effector cells, can be administered as a suspension of cells in a physiologically acceptable solution. The number of cells administered can be in the order of 10⁸-10⁹ but will vary depending on the therapeutic purpose. In general, the amount will be sufficient to obtain localization at the target cell, e.g., a tumor cell expressing PSMA, and to effect cell killing by, e.g., phagocytosis. Routes of administration can also vary.

Therapy with target-specific effector cells can be performed in conjunction with other techniques for removal of targeted cells. For example, anti-tumor therapy using the compositions (e.g., human antibodies, multispecific and bispecific molecules) of the invention and/or effector cells armed with these compositions can be used in conjunction with chemotherapy. Additionally, combination immunotherapy may be used to direct two distinct cytotoxic effector populations toward tumor cell rejection. For example, anti-PSMA antibodies linked to anti-Fc-gammaRI or anti-CD3 may be used in conjunction with IgG- or IgA-receptor specific binding agents.

Bispecific and multispecific molecules of the invention can also be used to modulate FcγR or FcαR levels on effector cells, such as by capping and elimination of receptors on the cell surface. Mixtures of anti-Fc receptors can also be used for this purpose.

The compositions (e.g., human antibodies, multispecific and bispecific molecules) of the invention which have complement binding sites, such as portions from IgG1, -2, or -3 or IgM which bind complement, can also be used in the presence of complement In one embodiment, *ex vivo* treatment of a population of cells comprising target cells with a binding agent of the invention and appropriate effector cells can be supplemented by the addition of complement or serum containing complement. Phagocytosis of target cells coated with a binding agent of the invention can be improved by binding of complement proteins. In another embodiment target cells coated with the compositions (e.g., human antibodies, multi specific and bispecific molecules) of the invention can also be lysed by complement.

The compositions (e.g., human antibodies, multispecific and bispecific molecules) of the invention can also be administered together with complement. Accordingly, within the scope of the invention are compositions comprising human antibodies, multispecific or bispecific molecules and serum or complement. These compositions are advantageous in that the complement is located in close proximity to the human antibodies, multispecific or bispecific molecules. Alternatively, the human antibodies, multispecific or bispecific molecules of the invention and the complement or serum can be administered separately.

Also within the scope of the invention are kits comprising the compositions (e.g., human antibodies, multispecific and bispecific molecules) of the invention and instructions for use. The kit can further contain a least one additional reagent, such as complement, or one or more additional human antibodies of the invention (e.g., a human antibody having a complementary activity which binds to an epitope in PSMAantigen distinct from the first human antibody).

In other embodiments, the subject can be additionally treated with an agent that modulates, e.g., enhances or inhibits, the expression or activity of Fcγ or Fcα receptors, by for example, treating the subject with a cytokine. Preferred cytokines for administration during treatment with the multispecific molecule include of granulocyte colony-stimulating factor (G-CSF), granulocyte- macrophage colony-stimulating factor (GM-CSF), interferon-γ (IFN-γ), and tumor necrosis factor (TNF).

The compositions (e.g., human antibodies, multispecific and bispecific molecules) of the invention can also be used to target cells expressing FcγR or PSMA, for example for labeling such cells. For such use, the binding agent can be linked to a molecule that can be detected. Thus, the invention provides methods for localizing *ex vivo* or *in vitro* cells expressing Fc receptors, such as FcγR, or PSMA. The detectable label can be, e.g., a radioisotope, a fluorescent compound, an enzyme, or an enzyme cofactor.

In one embodiment, the invention provides methods for detecting the presence of PSMA antigen in a sample, or measuring the amount of PSMA antigen, comprising contacting the sample, and a control sample, with a human monoclonal antibody, or an antigen binding portion thereof, which specifically binds to PSMA, under conditions that allow for formation of a complex between the antibody or portion thereof and PSMA. The formation of a complex is then detected, wherein a difference complex formation between the sample compared to the control sample is indicative the presence of PSMA antigen in the sample.

In still another embodiment, the invention provides a method for detecting the presence or quantifying the amount of Fc-expressing cells *in vivo* or *in vitro.* The method comprises (i) administering to a subject a composition (*e.g*., a multi- or bispecific molecule) of the invention or a fragment thereof, conjugated to a detectable marker, (ii) exposing the subject to a means for detecting said detectable marker to identify areas containing Fc-expressing cells. The present invention is further illustrated by the following examples which should not be construed as further limiting. The contents of all figures and all references, patents and published patent applications cited throughout this application are expressly incorporated herein by reference.

### EXAMPLES

### Example 1 Generation of Cmu targeted mice for the production of anti-PSMA human antibodies

### Construction of a CMD targeting vector

The plasmid pICEmu contains an EcoRI/XhoI fragment of the murine Ig heavy chain locus, spanning the mu gene, that was obtained from a Balb/C genomic lambda phage library (Marcu et al. Cell 22: 187,1980). This genomic fragment was subcloned into the XhoI/EcoRI sites of the plasmid pICEMI9H (Marsh et al; Gene 32, 481-485, 1984). The heavy chain sequences included in pICEmu extend downstream of the EcoRI site located just 3' of the mu intronic enhancer, to the XhoI site located approximately 1 kb downstream of the last transmembrane exon of the mu gene; however, much of the mu switch repeat region has been deleted by passage in *E. coli.*

The targeting vector was constructed as follows (see Figure 1). A 1.3 kb HindIII/SmaI fragment was excised from pICEmu and subcloned into HindIII/SmaI digested pBluescript (Stratagene, La Jolla, CA). This pICEmu fragment extends from the HindIII site located approximately 1 kb 5' of Cmul to the SmaI site located within Cmul. The resulting plasmid was digested with SmaI/SpeI and the approximately 4 kb SmaI/XbaI fragment from pICEmu, extending from the SmaI site in Cmul 3' to the XbaI site located just downstream of the last Cmu exon, was inserted, The resulting plasmid, pTAR1, was linearized at the SmaI site, and a neo expression cassette inserted. This cassette consists of the neo gene under the transcriptional control of the mouse phosphoglycerate kinase (pgk) promoter (XbaI/TaqI fragment; Adra et al. (1987) Gene 60: 65-74) and containing the pgk polyadenylation site (PvuII/HindIII fragment; Boer et al. (1990) Biochemical Genetics 28: 299-308). This cassette was obtained from the plasmid pKJ1 (described by Tybulewicz et al. (1991) Cell 65: 1153-1163) from which the neo cassette was excised as an EcoRI/HindIII fragment and subcloned into EcoRI/HindIII digested pGEM-7Zf (+) to generate pGEM-7 (KJ1). The neo cassette was excised from pGEM-7 (KJ1) by EcoRI/SalI digestion, blunt ended and subcloned into the SmaI site of the plasmid pTAR1, in the opposite orientation of the genomic Cmu sequences. The resulting plasmid was linearized with Not I, and a herpes simplex virus thymidine kinase (tk) cassette was inserted to allow for enrichment of ES clones bearing homologous recombinants, as described by Mansour et al. (1988) Nature 336: 348-352. This cassette consists of the coding sequences of the tk gene bracketed by the mouse pgk promoter and polyadenylation site, as described by Tybulewicz et al. (1991) Cell 65: 1153-1163. The resulting CMD targeting vector contains a total of approximately 5.3 kb of homology to the heavy chain locus and is designed to generate a mutant mu gene into which has been inserted a neo expression cassette in the unique SmaI site of the first Cmu exon. The targeting vector was linearized with PvuI, which cuts within plasmid sequences, prior to electroporation into ES cells.

### Generation and analysis of targeted ES cells

AB-1 ES cells (McMahon, A. P. and Bradley, A., (1990) Cell 62: 1073-1085) were grown on mitotically inactive SNL76/7 cell feeder layers (ibid.) essentially as described (Robertson, E. J. (1987) in Teratocarcinomas and Embryonic Stem Cells: a Practical Approach (E. J. Robertson, ed.) Oxford: IRL Press, p. 71-112). The linearized CMD targeting vector was electroporated into AB-1 cells by the methods described Hasty *et al.* (Hasty, P. R et al. (1991) Nature 350: 243-246). Electroporated cells were plated into 100 mm dishes at a density of 1-2 x 10⁶ cells/dish. After 24 hours, G418 (200 micrograms/ml of active component) and FIAU (5 x 10⁻⁷ M) were added to the medium, and drug-resistant clones were allowed to develop over 8-9 days. Clones were picked, trypsinized, divided into two portions, and further expanded. Half of the cells derived from each clone were then frozen and the other half analyzed for homologous recombination between vector and target sequences.

DNA analysis was carried out by Southern blot hybridization. DNA was isolated from the clones as described Laird *et al.* (Laird, P. W. et al., (1991) Nucleic Acids Res. 19: 4293). Isolated genomic DNA was digested with SpeI and probed with a 915 bp SacI fragment, probe A (see Figure 1), which hybridizes to a sequence between the mu intronic enhancer and the mu switch region. Probe A detects a 9.9 kb SpeI fragment from the wild type locus, and a diagnostic 7.6 kb band from a mu locus which has homologously recombined with the CMD targeting vector (the neo expression cassette contains a SpeI site). Of 1132 G418 and FIAU resistant clones screened by Southern blot analysis, 3 displayed the 7.6 kb SpeI band indicative of homologous recombination at the mu locus. These 3 clones were further digested with the enzymes BglI, BstXI, and EcoRI to verify that the vector integrated homologously into the mu gene. When hybridized with probe A, Southern blots of wild type DNA digested with BgII, BstXI, or EcoRI produce fragments of 15.7, 7.3, and 12.5 kb, respectively, whereas the presence of a targeted mu allele is indicated by fragments of 7.7, 6.6, and 14.3 kb, respectively. All 3 positive clones detected by the SpeI digest showed the expected BgII, BstXI, and EcoRI restriction fragments diagnostic of insertion of the neo cassette into the Cmul exon.

### Generation of mice bearing the mutated mu gene

The three targeted ES clones, designated number 264, 272, and 408, were thawed and injected into C57BL/6J blastocysts as described by Bradley (Bradley, A. (1987) in Teratocarciaomas and Embryonic Stem Cells: a Practical Approach. (E. J. Robertson, ed.) Oxford: IRL Press, p. 113-151). Injected blastocysts were transferred into the uteri ofpseudopregnant females to generate chimeric mice representing a mixture of cells derived from the input ES cells and the host blastocyst. The extent of ES cell contribution to the chimera can be visually estimated by the amount of agouti coat coloration, derived from the ES cell line, on the black C57BL/6J background. Clones 272 and 408 produced only low percentage chimeras (i.e. low percentage of agouti pigmentation) but clone 264 produced high percentage male chimeras. These chimeras were bred with C57BL/6J females and agouti offspring were generated, indicative of germline transmission of the ES cell genome. Screening for the targeted mu gene was carried out by Southern blot analysis of BgII digested DNA from tail biopsies (as described above for analysis ofES cell DNA). Approximately 50% of the agouti offspring showed a hybridizing BglI band of 7.7 kb in addition to the wild type band of 15.7 kb, demonstrating a germline transmission of the targeted mu gene.

### Analysis of transgenic mice for functional inactivation of mu gene

To determine whether the insertion of the neo cassette into Cmu1 has inactivated the Ig heavy chain gene, a clone 264 chimera was bred with a mouse homozygous for the JHD mutation, which inactivates heavy chain expression as a result of deletion of the JH gene segments (Chen et al, (1993) Immunol. 5: 647-656). Four agouti offspring were generated. Serum was obtained from these animals at the age of 1 month and assayed by ELISA for the presence of murine IgM. Two of the four offspring were completely lacking IgM (see Table 1). Genotyping of the four animals by Southern blot analysis of DNA from tail biopsies by BglI digestion and hybridization with probe A (see Figure 1), and by StuI digestion and hybridization with a 475 bp EcoRI/StuI fragment (ibid.) demonstrated that the animals which fail to express serum IgM are those in which one allele of the heavy chain locus carries the JHD mutation, the other allele the Cmu1 mutation. Mice heterozygous for the JHD mutation display wild type levels of serum Ig. These data demonstrate that the Cmul mutation inactivates expression of the mu gene.

**TABLE 1**

| **Mouse** | **Serum IgM (micrograms/ml)** | **Ig H chain genotype** |
|---|---|---|
| 42 | <0.002 | CMD/JHD |
| 43 | 196 | +/JHD |
| 44 | <0.002 | CMD/JHD |
| 45 | 174 | +/JHD |
| 129xBL6F1 | 153 | +/+ |
| JHD | <0.002 | JHD/JHD |

Table 1 shows the levels of serum IgM, detected by ELISA, for mice carrying both the CMD and JHD mutations (CMD/JHD), for mice heterozygous for the JHD mutation (+/JHD), for wild type (129Sv x C57BL/6J)F1 mice (+/+), and for B cell deficient mice homozygous for the JHD mutation (JHD/JHD).

### Example 2 Generation of HCO12 transgenic mice for the production of anti-PSMA human antibodies

### The HCO12 human heavy chain transgene

The HCO12 transgene was generated by coinjection of the 80 kb insert of pHC2 (Taylor et al., 1994, Int. Immunol., 6: 579-591) and the 25 kb insert of pVx6. The plasmid pVx6 was constructed as described below.

An 8.5 kb HindIII/SalI DNA fragment, comprising the germline human VH1-18 (DP-14) gene together with approximately 2.5 kb of 5' flanking, and 5 kb of 3' flanking genomic sequence was subcloned into the plasmid vector pSP72 (Promega, Madison, WI) to generate the plasmid p343.7.16. A 7 kb BamHI/HindIII DNA fragment, comprising the germline human VH5-51 (DP-73) gene together with approximately 5 kb of 5' flanking and 1 kb of 3' flanking genomic sequence, was cloned into the pBR322 based plasmid cloning vector pGP1f (Taylor et al. 1992, Nucleic Acids Res. 20: 6287-6295), to generate the plasmid p251f. A new cloning vector derived from pGP1f, pGP1k (SEQ ID NO:13), was digested with EcoRV/BamHI, and ligated to a 10 kb EcoRV/BamHI DNA fragment, comprising the germline human VH3-23 (DP47) gene together with approximately 4 kb of 5' flanking and 5 kb of 3' flanking genomic sequence. The resulting plasmid, p112.2RR.7, was digested with BamHI/SalI and ligated with the 7 kb purified BamHI/SaII insert of p251f. The resulting plasmid, pVx4, was digested with XhoI and ligated with the 8.5 kb XhoI/SalI insert of p343.7.16.

A clone was obtained with the VH1-18 gene in the same orientation as the other two V genes. This clone, designated pVx6, was then digested with NotI and the purified 26 kb insert coinjected--together with the purified 80 kb NotI insert of pHC2 at a 1:1 molar ratio--into the pronuclei of one-half day (C57BL/6J x DBA/2J)F2 embryos as described by Hogan *et al.* (B. Hogan et al., Manipulating the Mouse Embryo, A Laboratory Manual, 2nd edition, 1994, Cold Spring Harbor Laboratory Press, Plainview NY). Three independent lines of transgenic mice comprising sequences from both Vx6 and HC2 were established from mice that developed from the injected embryos. These lines are designated (HCO12)14881, (HCO12)15083, and (HCO12)15087. Each of the three lines were then bred with mice comprising the CMD mutation described in Example 1, the JKD mutation (Chen et al. 1993, EMBO J. 12: 811-820), and the (KCoS)9272 transgene (Fishwild et al. 1996, Nature Biotechnology 14: 845-851). The resulting mice express human immunoglobulin heavy and kappa light chain transgenes in a background homozygous for disruption of the endogenous mouse heavy and kappa light chain loci.

### Example 3 Production of Human Monoclonal Antibodies and Bispecifics Against PSMA

Human anti-PSMA monoclonal antibodies were generated by immunizing the HCO7 and HCO12 strains of HuMAb mice with PSMA antigen purified from LNCaP cells which express PSMA and/or a crude membrane preparation from LNCaP cells.

HCO7 HuMAb mice were generated as described in U.S. Patent Nos. 5,770,429 and 5,545,806, the entire disclosures of which are hereby incorporated by reference.

In particular, HCO7 and HCO12 mice were initially immunized with antigens emulsified in Complete Freund's Adjuvant. In subsequent immunizations, antigens were mixed with Incomplete Freund's Adjuvant. Finally, intravenous administration of antigen prior to splenectomy was performed with purified antigen, and no adjuvant. Mice were immunized every 2-3 weeks. After the third and following immunizations, the human IgG anti-PSMA titer was determined by ELISA (as described below). Mice that developed an auti-PSMA IgG response were administered purified antigen i.v. for three days or both three and four days prior to splenectomy. The spleens from responding mice were harvested and dispersed into single cells.

To generate hybridomas producing arrti-PSMA antibodies, splenocytes from mice with plasma containing anti-PSMA antibodies were fused with P3X63-Ag8.653 cells (deposited with the ATCC under designation ATCC CRL 1580 nonsecreting mouse myeloma cells) and PEG. After hybridomas grew out (about 10-14 days) each well containing hybridomas was screened for the production of human IgG using an anti-human IgG ELISA.

Positive hybridomas were screened for and selected based on the following properties: (1) production of human IgGlκ antibodies, (2) binding to purified PSMA, and (3) binding to PSMA-expressing tumor cells. DNA sequence analysis confirmed that the anti-PSMA antibodies were encoded by V(D)J rearrangements of the human immunoglobulin transgenes.

Briefly, a solid-phase ELISA based assay was employed for the analysis of human anti-PSMA antibodies. Immunoaffinity purified PSMA from LNCaP cells was coated onto Maxi-Sorp 96-well microtiter plates (Nunc, Rochester, NY) and incubated overnight at 4°C. The plates were then washed with PBS-0.2%Tween-20 and blocked with 5% bovine serum albumin in PBS for 1 hour at room temperature. Supematants from the anti-PSMA producing hybridomas (50 µl) or purified antibody (1-5 µg/ml in PHS) were added to the wells and incubated for 2 hours at room temperature. The plates were washed with PBS/Tween as above and then incubated with 50 µl of HRP-conjugated rabbit-anti-human IgG (1:5000, Jackson Immunoresearch, West Grove, PA) for 1 hour atroom temperature. After washing, 100 µl of ABTS (150 mg 2,2'-azino-bis (3-ethylbenzthiazoline-6-sulfonic acid in 500 ml of 0.1 M citric acid, pH 4.35)/H₂O₂ (10 µl 30% H₂O₂ per 10 ml of ABTS solution) chromagen/substrate solution was added to each well and the samples read with a microplate reader at a wavelength of 405nm.

Hybridomas which produced human IgG that bound PSMA by ELISA were further characterized by ELISA and flow cytometry for isotype, binding to tumor cells expressing PSMA, and lack of binding to PSMA-negative cells, as described below. Hybridomas with supernatants exhibiting these properties were subcloned, and their antibodies further characterized after purification.

Monoclonal antibodies were isolated from a Cellmax bioreactor (Cellco, Laguna Hills, CA) using HyQ-CCM1 medium containing 1 to 5% Fetalclone (Hyclone, Logan, UT) and purified by chromatography on a Protein G-agarose column according to the manufacturer's specifications (KPL, Gaithersburg, MD).

Purified monoclonal antibodies were extensively dialyzed against 0.3 M sodium carbonate buffer, pH 9.5, for labeling with fluorescence isothiocyanate (FITC). A stock FITC solution was prepared by dissolving 1 mg solid FITC in 1 ml of DMSO. Stock FITC was added dropwise with constant mixing in an amount to provide 50 µg FITC per mg of antibody protein. Following the addition of FITC, the solution was incubated in the dark for 1-3 hours at room temperature. FITC labeled antibodies were isolated by gel filtration on a Sephadex G-10 column equilibrated in PBS.

Several hybridomas that were screened produced human IgG1κ antibodies that specifically bind to PSMA as assessed by ELISA and flow cytometry (e.g., 4A3, 7F12, 8A11, 8C12 and 16F9). Monoclonal antibodies purified from hybridoma 8C12 supernatant that showed significant binding to PSMA as detected by ELISA were characterized as having a specific binding affinity of ≥10⁻⁹.

Bispecific molecules designated 14.1 x 8C12 (shown in Figure 3) and 14A8 x 8C12 were made by chemical conjugation of the Fab'₂ fragments from the human anti-CD89 antibody 14.1 or a subclone of the 14.1 antibody, designated 14A8, respectively, and the human anti-PSMA antibody, 8C12, via disulfide bonds using standard cross-linking procedures.

### Example 4 Characterization of Human Monoclonal Antibodies Against PSMA

### I. Binding of human anti-PSMA antibodies to tumor cells

Monoclonal antibodies purified from hybridoma supernatants that showed significant binding to PSMA as detected by ELISA (e.g., 4A3, 7F12, 8A11, 8C12 and 16F9) were further tested for the ability to bind to tumor cells that express high levels of PSMA.
The binding characteristics of human anti-PSMA specific antibodies were studied by solid phase ELISA using plasma membrane fractions derived from both LNCaP and PC3 cells. To prepare cell membrane fractions from LNCaP and PC3 cells, cells were scraped from plastic dishes, washed extensively in PBS, resuspended in 10 volumes of deionized water, and homogenized by three strokes with a Dounce homogenizer. The membrane fraction was isolated by centrifugation at 15,000 x g for 45 minutes and the pellet resuspended in PBS. The protein concentration of the membrane pellet was determined using the Pierce (Rockford, IL) BSA kit.

LNCaP and PC3 membrane fractions were serially diluted in 96-well plates and air dried. The plates were blocked with 5% BSA, and treated with 5µg/ml human anti-PSMA antibody in PBS for 1 hour, prior to detection using standard ELISA procedures as described above. The results presented in Figure 4 show results for the independent human monoclonal antibodies. These antibodies demonstrate high specificity for LNCaP cell membranes over a range of antigen concentrations, whereas there is little or no antibody binding above background to PC3 cell membranes.

In addition, human anti-PSMA antibody binding to live LNCaP and PC3 tumor cells was studied by flow cytometry. Briefly, tumor cells were freshly harvested from tissue culture and a single cell suspension was prepared. Approximately 1 million cells were resuspended in PBS containing 0.5% BSA and incubated with 50-200 µg/ml of FITC-labeled human anti-PSMA antibody for 30 minutes on ice. The cells were washed twice with PBS containing 0.1% BSA, 0.01% NaN3, and analyzed for fluorescent staining on a FACSCalibur cytometer (Becton-Dickinson, San Jose, CA) with CellQuest acquisition software. As shown in Figure 5, human anti-PSMA antibodies 4A3, 7F12, 8A11, 8C12 and 16F9 demonstrated strong specific binding to live LNCaP cells expressing PSMA. In contrast, negative staining was observed with PC3 cells, or when an isotype matched irrelevant human antibody was used with LNCaP.

Bispecific molecule 14.1 x 8C12 was also tested for the ability to bind to PSMA-expressing LNCaP cells (Figure 6) and CD89-expressing U937 cells (Figure 7) using similar assays. As shown in Figure 6 and 7, the bispecific molecule binds both LNCaP and U937 cells in a dose-dependent fashion.

### II. Binding specificity of human anti-PSMA antibodies

The binding specificity of anti-PSMA antibodies 4A3, 7F12, 8A11, 8C12 and 16F9 was further studied by immunoprecipitation of protein lysates from LNCaP cells. Briefly, a detergent lysate of LNCaP cells was prepared by adding PBS containing 1 % NP-40 to LNCaP cells, incubating for 1 hour, followed by centrifugation to remove particulate material. The lysate was pre-cleared by adding 150 µg irrelevant human IgGl per milliliter of lysate, incubating for 1 hour at room temperature, followed by the addition of 150 µl of packed Protein G-Sepharose beads per milliliter of lysate. The supernatant fraction was used after centrifugation to remove the beads.

Aliquots (100µl) of the pre-cleared LNCaP cell lysate were mixed with 5 µg of an individual human anti-PSMA, antibody and incubated overnight at 4°C. The beads were washed extensively with PBS, followed by the addition of SDS sample buffer. The bound immune complexes were subjected to SDS polyacrylamide gel electrophoresis and transferred to PVDF membranes for Western blot analysis. Membranes were blocked overnight in TBS/5% noufat milk and incubated with the murine linear PSMA sequence epitope specific antibody 4D8, 5 µg/ml in TBS, for 1 hours. The blots were washed 5 times with TBS containing 0.5% Tween-20 (TBS-T) and incubated with HRP-conjugated goat anti-mouse IgG (1:5000). After 5 washes with TBS-T, the blots were developed using the LumiGhO chemilluminescent substrate kit (KPL, Gaithersburg, MD) and visualized by exposure to x-ray film.

The results are shown in Figure 8. The identities of PSMA and PSM', an alternative splice variant missing the first 57 amino acids from the N-terminus, are indicated by the arrows. Lane 1 shows Western blot reactivity of PSMA and PSM' present in LNCaP cell lysate. Lane 2 shows results from immunoprecipitation with an isotype matched (IgG1) irrelevant human antibody. Lanes 3 through 7 show results from immunoprecipitation with antibodies 4A3, 7F12, 8A11, 8C12 and 16F9, respectively. In each case, intense bands corresponding to the both PSMA and PSM' are observed, indicating that these antibodies bind to protein epitopes present within the extracellular domain of the protein, a region spanning amino acid residues 44-750.

Antibody binding to native and heat denatured PSMA was tested to determine the importance of protein confirmation on binding specificity. An aliquot of immunoaffinity purified PSMA from LNCaP cells (40 µg/ml in PBS) was heat denatured by boiling for 10 minutes, and cooled on ice. The heat denatured PSMA and an identical aliquot of non-denatured PSMA were diluted 1:4 in PBS, added to the wells of a 96-well Maxi-Sorp plate (Nunc), and incubated overnight at 4°C. After coating, plates were blocked with 5% BSA in PBS for 1 hour, washed in PBS, and subjected to a sandwich ELISA using the human anti-PSMA antibodies. The murine anti-PSMA monoclonal antibody 7E11, specific for an epitope comprised of the first 6 amino acids from the N-terminus of the protein, was used as a positive control. As shown in Figure 9, heat denaturation had no impact on 7E11 antibody binding, consistent with its recognition of a linear sequence epitope. In contrast to these results, human anti-PSMA antibodies 4A3, 7F12, 8A11, 8C12, and 16F9 all strongly bound to native purified PSMA, while heat denaturation of PSMA virtually abolished antibody binding. These results indicate that a native protein confirmation is required for human anti-PSMA antibody binding. Consistent with this result, antibodies 4A3, 7F12, 8A11, 8C12, and 16F9 were ineffective in detecting PSMA in a Western blot analysis.

### III. Antibody binding competition studies

Antibody binding competition studies were conducted using flow cytometry analysis to determine whether the individual human anti-PSMA antibodies bound to a similar or distinct epitopes on PSMA. In these experiments, the ability of unlabeled anti-PSMA antibodies to block binding of FITC-labeled anti-PSMA antibodies to LNCaP cells was tested. The intensity of FITC labeling of LNCaP cells was determined by flow cytometry.

Aliquots of approximately 1 million LNCaP cells were pre-treated with 200 µg/ml purified human anti-PSMA antibody or irrelevant human IgG1 antibody in PBS for 1 hour on ice. The cells were extensively washed and further incubated with 50 µg/ml FITC-conjugated human anti-PSMA antibody (or in some experiments FITC-conjugated murine PSMA specific antibody, e.g., 1G9, 3C6 and 4D4) for 1 hour on ice. After washing, the cells were stained with 10 µg/ml propidium iodide and analyzed by flow cytometry on a FACSCalibur with CellQuest software.

As shown in Figure 10, strong binding of the FITC-labeled human anti-PSMA antibodies was observed in each case with LNCaP cells pre-treated with irrelevant human IgG1. In contrast, pre-treatment with individual human anti-PSMA antibodies resulted in substantial inhibition of the subsequent binding of FITC-labeled human anti-PSMA antibody in each case. Slight variation in the extent of binding inhibition was observed with different antibody pairings. For example, 8C12 effectively inhibits 4A3, 8A11 and 8C12 binding, but has much less of an effect on 7F12. Taken together, these data suggest that the competitive binding behavior of 7F12 and 16F9 with the other human anti-PSMA antibodies are most similar. Overall, it appears that the individual human anti-PSMA antibodies recognized slightly different, but closely distributed conformational epitopes on PSMA.

Antibody competition studies were also conducted using a small panel of murine PSMA-specific antibodies, designated 1G9, 3C6 and 4D4, which are also directed toward protein conformational epitopes, to determine if the human anti-PSMA antibodies recognize epitopes in common with the murine antibodies. As shown in Figure 11, pre-treatment of LNCaP cells with individual human anti-PSMA antibodies, followed by labeling with either FITC-murine 4D4 and 1G9, resulted in little or no apparent inhibition. In contrast, significant inhibition of FITC-murine 3C6 binding was observed by human anti-PSMA antibodies 7F12, 8A11, 8C12, and 16F9, indicating that each of these antibodies binds to a similar or closely distributed epitope as that recognized by 3C6. No inhibition of FITC-labeled murine conformational antibodies was observed with human anti-PSMA antibody 4A3, suggesting that it binds to an epitope distinct from either murine 1G9, 3C6, or 4D4.

### Example 5 Activity of human anti-PSMA antibodies

### I. Inhibition of human tumor cell growth using human anti-PSMA antibodies and bispecifics

Anti-PSMA antibodies and bispecifics that show specific binding to PSMA, as demonstrated above, can be tested for the ability to inhibit the growth of human tumor cells expressing PSMA. Briefly, purified antibodies can be incubated with the tumor cells under normal growth conditions, and cell density can be measured by crystal violet staining.

### II. Antibody dependent cell-mediated cytotoxicity (ADCC) activity of human anti-PSMA antibodies and bispecifics

Bispecific molecules 14.1 x 8C12 (shown in Figure 3) and 14A8 x 8C12, as well as monoclonal antibody 8C12, were tested for polymorphonuclear cell-mediated ADCC ldlling of labeled PSMA-expressing tumor cells.

In particular, mononuclear cells (monocytes and neutrophils), as well as whole blood, were isolated from healthy donors and incubated with ⁵¹Cr labeled PSMA expressing tumor cells in the presence ofbispecific molecule 14.1 x 8C12. After approximately 4 hours, the culture supernatant from the wells was harvested and ⁵¹Cr release measured on a gamma counter. The percent specific lysis was determined according the following formula: (experimental CPM - target leak CPM)/(detergent lysis CPM - target leak CPM) × 100%. The results, shown in Figures 12A, 13A and 14A, demonstrate that 14.1 x 8C12 mediates dose dependent lysis of tumor cells by monocytes and neutrophils and whole blood, respectively, as compared to a control antibody.

Mononuclear cells and whole blood were also incubated with ⁵¹Cr labeled LNCaP tumor cells in the presence of either bispecific molecule 14A8 x 8C12 or monoclonal antibody 8C12 (Figures 12B,12C, 13B and 14B). LNCaP cells were labeled with 100 µCi of ⁵¹Cr for 1 hour at 37°C (5% CO₂) prior to incubation with mononuclear cells and whole blood, along with various concentrations of bispecific or monoclonal antibody. After an incubation of 16 hours, the supernatant was harvested and analyzed for radioactivity as described above.

### Monocyte Induced ADCC

As shown in Figure 12A, bispecific molecule 14.1 × 8C12 mediated cell killing of tumor cells expressing PSMA by monocytes in a dose dependent fashion. Addition of 50 µg/ml of 14.1 Fab'₂ completely blocked ADCC of the tumor cells by 1 µg/ml of the bispecific molecule 14.1 × 8C12, demonstrating that targeted cell killing was mediated exclusively by CD89 on the effector cells. As shown in Figure 12B, bispecific molecule 14A8 x 8C12 and monoclonal antibody 8C12 also mediated dose dependent lysis by monocytes of LNCaP tumor cells. Moreover, the addition of excess 14A8 F(ab)'₂ completely inhibited ADCC of the tumor cells by bispecific molecule 14A8 x 8C12 as compared to H22 F(ab)'₂ (humanized anti-FcγRI), indicating that targeted cell killing was mediated through CD89 (see Figure 12 C).

### Neutrophil Induced ADCC

As shown in Figure 13A, bispecific molecule 14.1 × 8C12 mediated cell killing of tumor cells expressing PSMA by neutrophils in a dose dependent fashion. Addition of 25 µg/ml of 14.1 Fab'₂ significantly blocked ADCC of the tumor cells by the bispecific molecule, demonstrating that targeted cell killing was mediated specifically by CD89 binding to the effector cells. As shown in Figure 13B, bispecific molecule 14A8 x 8C12 also mediated dose dependent lysis by neutrophils of LNCaP tumor cells. The addition of excess 14A8 F(ab)'₂ completely inhibited ADCC of the tumor cells by 14A8 x 8C12 as compared to H22 F(ab)'₂ (humanized anti-FcγRI), indicating that targeted cell killing was mediated through CD89.

### Whole Blood Induced ADCC

As shown in Figure 14A, bispecific molecule 14.1 × 8C12 mediated cell killing of tumor cells expressing PSMA by whole-blood in a dose dependent fashion. Addition of 25 µg/ml of 14.1 Fab'₂ significantly blocked ADCC of the tumor cells by the bispecific molecule, again demonstrating that targeted cell killing was mediated specifically by CD89 binding to the effector cells. Similarly, as shown in Figure 14B, bispecific molecule 14A8 x 8C12 also mediated dose dependent lysis by whole blood of LNCaP tumor cells. The addition of excess 14A8 F(ab)'₂ completely inhibited ADCC of the tumor cells by 14A8 x 8C12 as compared to H22 F(ab)'₂ (bumaized anti-FcγRI), indicating that targeted cell killing was mediated through CD89.

### III. Human anti-PSMA antibodies and bispecifics mediate phagocytosis and killing of tumor cells expressing PSMA in the presence of human effector cells

Bispecific molecule 14.1 x 8C12 and 14A8 x 8C12, as well as monoclonal antibody 8C12, were tested for their ability to mediate phagocytosis of labeled PSMA-expressing tumor cells (LnCAP cells) alone, as well as in the presence of excess 14.1 (human anti-FcαR) Fab'₂ antibody or excess H22 (humanized anti-FcγRI) Fab'₂ antibody as a control.

Briefly, bispecific-mediated phagocytosis of LnCAP cells by monocyte derived macrophages (MDM) was examined by a modification of the method described Munn et al. (1990) J. Exp. Med. 172:231-237. Monocytes, purified from normal adult source leukopacs (ABI), were differentiated in 24-well plates (1 x 10⁶/ml) in macrophage serum free medium (Gibco, Grand Island, NY) supplemented with 10% FBS and 10ng/ml of M-CSF for 7-12 days LnCAP cells were labeled with the lipophilic red fluorescent dye, PKH 26 (Sigma, St. Louis, MO). The labeled LnCAP cells were added to the wells containing MDM in the absence or presence of bispecific antibody (or control antibody) and incubated at 37° C for 5-24 hours (5% CO₂). MDM and non-phagocytized LnCAP cells were recovered with trypsin, and stained with a FITC-labeled anti-CD33 mAb (251) and an anti-CD14 mAb (AML-2-23) for 1 hour on ice (4°C). Cells were washed and analyzed by two color fluorescence using the FACScan. Percent phagocytosis was calculated as the number of dual-positive target cells (ingested by MDM) divided by the total number of target cells x 100%.

As shown in Figure 15, bispecific molecule 14.1 x 8C12 mediated increasing specific phagocytosis of tumor cells in a dose-dependent fashion. Addition of 14.1 Fab'₂ significantly blocked phagocytosis of the tumor cells by the bispecific molecule, again demonstrating that targeted phagocytosis was mediated specifically by CD89 binding to the effector cells. Similarly, as shown in Figure 16, bispecific molecule 14A8 x 8C12 and monoclonal antibody 8C12 also mediated phagocytosis of LNCaP cells in a dose dependent fashion. Figure 17 shows that 14A8 x 8C12 mediated phagocytosis of LNCaP tumor cells was mediated through CD89, as it was inhibited by the addition of excess 14A8 F(ab)'₂, as compared to H22 F(ab)'₂ (humaized anti-FcγRI) (see inset, Figure 17).

The foregoing Examples demonstrate the generation of human monoclonal antibodies and bispecifics that specifically react with high affinity to PSMA. These antibodies and bispecifics appear to recognize native conformational protein epitopes present in the extracellular domain of the molecule, rather than epitopes defined by a linear amino acid sequence. In addition, the human anti-PSMA antibodies and bispecific molecules thereof mediate cell killing and phagocytosis in the presence of effector cells against human tumor cells expressing high levels of PSMA. These results support the conclusion that the fully human monoclonal antibodies against PSMA and fragments and bispecific molecules thereof of the present invention will be useful for the diagnosis and treatment of PSMA related disorders.

### Equivalents

Those skilled in the art will recognize, or be able to ascertain using no more than routine experimentation, many equivalents of the specific embodiments of the invention described herein. Such equivalents are intended to be encompassed by the following claims.
1. G.Kobler and Milstein C. (1975) Continuous cultures of fused cells secreting antibody of predefined specificity. Nature 256: 495-497.
2. G.L. Boulianne. HozumN., and Shulman MJ. (1984) Production of functional chimeric mouse/human antibody. Nature 312:643-646.
3. P.T. Jones. Dear P.H., Foote J., Neuberger M.S., and Winter G. (1989) Replacing the complementarity-determining regions in a human antibody with those of a mouse. Nature 321: 522-525.
4. J.D. Marks et al.(1991) By-passing Immunization Human antibodies from V-gene libraries displayed on phage. J. Mol. Biol. 222: 581-597.
5. N. Lonberg, et al. 1994. Antigen-specific human antibodies from mice comprising four distinct genetic modifications. Nature 368(6474): 856-859.
6. G.Gafie, Howe S.C., Butcher M.C. C. W., and Howard H.C. (1997) Antibodies to major histocompatibility antigens produced by hybrid cell lines. Nature 266:550-552.
7. Heston, W.D. (1996) Urologe-Ausgabe A. 35:400-407.
8. Gregorakis, A.K. et al. (1998) Prostate-specific membrane antigen: current and future utility. Seminars in Urologic Oncology 16:2-12.
9. Barren, R.J. 3rd et al. (1998) Method for identifying prostate cells in semen using flow cytometry. Prostate 36:181-188.
10. Murphy, G.P. (1998) Current evaluation of the tissue localization and diagnostic utility of prostate specific membrane antigen. Cancer 83:2259-2269.
11. Silver, D.A. (1997) Prostate-specific membrane antigen expression in normal and malignant human tissues. Clinical Cancer Research 3:81-85.
12. O'Keefe, D.S. et al. (1998) Mapping, genomic organization and promoter analysis of the human prostate-specific membrane antigen gene. Biochim. Biophys. Acta. 1443:113-127.

## Claims

1. An isolated human monoclonal antibody, or an antigen binding portion thereof, that specifically binds to PSMA, wherein the antibody or antigen binding portion thereof has one or more of the following characteristics:
a) a binding affinity constant to PSMA of at least about 10⁷ M⁻¹;
b) the ability to opsonize a cell expressing PSMA; or
c) the ability to inhibit growth or to mediate cytolysis of a cell expressing PSMA in the presence of human effector cells at a concentration of about 10 µg/ml or less *in vitro.*

2. The isolated human antibody of claim 1, or an antigen binding portion thereof, having an isotype selected from the group consisting of IgG1, IgG2, IgG3, IgG4, IgM, IgA1, IgA2, IgAsec, IgD, and IgE.

3. The isolated human antibody of claim 1, or an antigen binding portion thereof, which is an IgG1κ.

4. The isolated human antibody of claim 1, or an antigen binding portion thereof, wherein the cell expressing PSMA is a tumor cell.

5. The isolated human antibody of claim 4, or an antigen binding portion thereof, wherein the cell expressing PSMA is a prostate cell.

6. The isolated human antibody of claim 1, or an antigen binding portion thereof, produced by a hybridoma which includes a B cell obtained from a transgenic non-human animal having a genome comprising a human heavy chain transgene and a human light chain transgene fused to an immortalized cell.

7. An isolated human monoclonal antibody, or antigen-binding portion thereof, which mediates cytolysis of cells expressing PSMA in the presence of human effector cells.

8. The isolated human antibody of claim 7, or an antigen binding portion thereof, which is capable of mediating cytolysis of cells expressing PSMA by human effector cells at an IC₅₀ of 1 x 10⁻⁷ M or less *in vitro.*

9. An isolated human monoclonal antibody, or antigen-binding portion thereof, which inhibits growth of cells expressing PSMA.

10. The isolated human antibody of claim 9, or an antigen binding portion thereof, which is capable of inhibiting growth of cells expressing PSMA at an IC₅₀ of 1 x 10⁻⁷ M or less *in vitro.*

11. A hybridoma comprising a B cell obtained from a transgenic non-human animal having a genome comprising a human heavy chain transgene and a light chain transgene fused to an immortalized cell, wherein the hybridoma produces a detectable amount of a human monoclonal antibody that specifically binds to PSMA.

12. The hybridoma of claim 11, wherein the human monoclonal antibody has one or more of the following characteristics:
a) a binding affinity constant to PSMA of at least about 10⁷ M⁻¹;
b) the ability to opsonize a cell expressing PSMA; or
c) the ability to inhibit growth or to mediate cytolysis of a cell expressing PSMA in the presence of human effector cells at a concentration of about 10 µg/ml or less *in vitro.*

13. A transgenic non-human animal which expresses a human monoclonal antibody that specifically binds to PSMA, wherein the transgenic non-human animal has a genome comprising a human heavy chain transgene and a human light chain transgene.

14. A method of producing a human monoclonal antibody that specifically binds to PSMA, comprising:
immunizing a transgenic non-human animal having a genome comprising a human heavy chain transgene and a human light chain transgene with PSMA or a cell expressing PSMA, such that antibodies are produced by B cells of the animal;
isolating B cells of the animal; and
fusing the B cells with myeloma cells to form immortal, hybridoma cells that secrete human monoclonal antibodies specific for PSMA.

15. A bispecific molecule comprising at least one first binding specificity for PSMA and a second binding specificity an Fc receptor.

16. The bispecific molecule of claim 15, wherein the Fc receptor is a human Fcγ RI or a human Fcα receptor.

17. The bispecific molecule of claim 15, which binds to the Fc receptor at a site which is distinct from the immunoglobulin binding site of the receptor.

18. An antibody or antigen-binding portion thereof according to any one of claims 1 to 10, which is conjugated to a cytotoxin.

19. An antibody or antigen-binding portion thereof according to claim 18, wherein the cytotoxin is selected from taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, abrin, ricin A, pseudomonas exotoxin and diphtheria toxin.

20. An antibody or antigen-binding portion thereof according to any one of claims 1 to 10, which is conjugated to a radioisotope.

21. An antibody or antigen-binding portion thereof according to any one of claims 1 to 10, which is conjugated to a therapeutic agent.

22. An antibody or antigen-binding portion thereof according to claim 22, wherein the therapeutic agent is selected from antimetabolites, alkylating agents, anthracyclines, antibiotics, anti-mitotic agents, growth factors, lymphokines, tumor necrosis factor and interferon-γ.

23. An antibody or antigen-binding portion thereof according to claim 22, wherein the antimetabolites are selected from methotrexate, 6-mercaptopurine, 6-thioguanine, cytarabine and 5-fluorouracil decarbazine, the alkylating agents are selected from mechlorethamine, thioepa chlorambucil, melphalan, carmustine (BSNU), lomustine (CCNU), cyclothosphamide, busulfan, dibromomannitol, streptozotocin, mitomycin C and cis-dichlorodiamine platinum (II) (DDP) cisplatin, the anthracyclines are selected from daunorubicin and doxorubicin, the antibiotics are selected from dactinomycin, bleomycin, mithramycin, and anthramycin (AMC), the antimitotic agents are selected from vincristine and vinblastine, the growth factors are selected from granulocyte macrophage colony stimulating factor (GM-CSF) and granulocyte colony stimulating factor (G-CSF) or the lymphokines are selected from interleukin-1 (IL-1), interleukin-2 (IL-2) and interleukin-6 (IL-6).

24. A composition comprising an isolated human monoclonal antibody or antigen-binding portion thereof of claim 1, and a pharmaceutically acceptable carrier.

25. A composition comprising a combination of two or more isolated human antibodies or antigen-binding portions thereof according to claim 1, wherein each of said antibodies or antigen-binding portions thereof binds to a distinct epitope of PSMA.

26. A method of inhibiting growth of a cell expressing PSMA, comprising contacting a cell expressing PSMA with an isolated human monoclonal antibody, or an antigen binding portion thereof, that specifically binds to PSMA, such that the growth of the cell expressing PSMA, is inhibited.

27. A method of inducing cytolysis of a cell expressing PSMA, comprising contacting a cell expressing PSMA with an isolated human monoclonal antibody, or an antigen binding portion thereof, that specifically binds to PSMA, in the presence of an effector cell, such that cytolysis of the cell expressing PSMA occurs.

28. Use of an isolated human monoclonal antibody, or an antigen binding portion thereof, that specifically binds to PSMA for the manufacture of a medicament for treating or preventing a disease **characterized by** aberrant expression of PSMA.

29. The method of claim 28, wherein the human monoclonal antibody is conjugated to a binding specificity for a Fc receptor.

30. The method of claim 28, wherein the human monoclonal antibody is conjugated to a cytotoxin.

31. The method of claim 28, wherein the disease is a cancer.

32. The method of claim 31, wherein the cancer is prostate cancer.

33. A method for detecting the presence of PSMA antigen, or a cell expressing PSMA, in a sample comprising:
contacting the sample, and a control sample, with a human monoclonal antibody, or an antigen binding portion thereof which specifically binds to PSMA, under conditions that allow for formation of a complex between the antibody or portion thereof and PSMA; and
detecting the formation of a complex,
wherein a difference complex formation between the sample compared to the control sample is indicative the presence of PSMA in the sample.

34. An expression vector comprising a nucleotide sequence encoding a variable and constant region of the heavy and light chains of a human monoclonal antibody, or an antigen binding portion thereof, that specifically binds to PSMA, wherein the antibody or antigen binding portion thereof has one or more of the following characteristics:
a) a binding affinity constant to PSMA of at least about 10⁷ M⁻¹;
b) the ability to opsonize a cell expressing PSMA; or
c) the ability to inhibit growth or to mediate cytolysis of a cell expressing PSMA in the presence of human effector cells at a concentration of about 10 µg/ml or less *in vitro.*

35. The isolated human antibody of claim 1, wherein the antibody or an antigen binding portion thereof binds to a protein epitope within the extracellular domain of the PSMA molecule.

36. An isolated nucleic acid molecule comprising a nucleotide sequence encoding an antibody or antigen binding portion according to any one of claims 1 to 10.
